(19) Europäisches Patentamt
European Patent Office
Office européen des brevets

(11) EP 1 548 866 A1

(12) EUROPEAN PATENT APPLICATION
published in accordance with Art. 158(3) EPC

(43) Date of publication:
**29.06.2005 Bulletin 2005/26**

(21) Application number: **03791250.8**

(22) Date of filing: **22.08.2003**

(51) Int Cl.[7]: **H01M 10/40**, H01M 2/16, C07C 217/08, C07C 311/48, C07D 233/56

(86) International application number:
**PCT/JP2003/010629**

(87) International publication number:
**WO 2004/021500 (11.03.2004 Gazette 2004/11)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **28.08.2002 JP 2002248004**
**27.05.2003 JP 2003149584**

(71) Applicant: **Nisshinbo Industries, Inc.**
**Chuo-ku, Tokyo 103-8650 (JP)**

(72) Inventors:
- **MARUO, Tatsuya**
  **c/o NISSHINBO INDUSTRIES, INC.**
  **Midori-ku, Chiba-shi, Chiba 267-0056 (JP)**
- **MARUKANE, Syoko**
  **c/o NISSHINBO INDUSTRIES, INC.**
  **Midori-ku, Chiba-sh i, Chiba 267-0056 (JP)**
- **MASUDA, Gen**
  **c/o NISSHINBO INDUSTRIES, INC.**
  **Midori-ku, Chiba-shi, Chiba 267-0056 (JP)**
- **SATO, Takaya c/o NISSHINBO INDUSTRIES, INC.**
  **Midori-ku, Chiba-shi, Chiba 267-0056 (JP)**

(74) Representative: **Stuart, Ian Alexander**
**Mewburn Ellis LLP**
**York House**
**23 Kingsway**
**London WC2B 6HP (GB)**

# (54) NONAQUEOUS ELECTROLYTE AND NONAQUEOUS-ELECTROLYTE SECONDARY BATTERY

(57) A nonaqueous electrolyte which contains an ionic liquid having general formula (1) below and a melting point not higher than 50°C, a compound which reductively decomposes at a more noble potential than the ionic liquid, and a lithium salt.

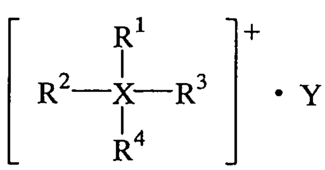

(1)

In formula (1), $R^1$ to $R^4$ are each independently an alkyl group of 1 to 5 carbons or an alkoxyalkyl group of the formula R'-O-$(CH_2)_n$- (R' being methyl or ethyl, and the letter n being an integer from 1 to 4), and any two from among $R^1$, $R^2$, $R^3$ and $R^4$ may together form a ring, with the proviso that at least one of $R^1$ to $R^4$ is an alkoxyalkyl group of the above formula. X is a nitrogen atom or a phosphorus atom, and Y is a monovalent anion.

EP 1 548 866 A1

## Description

### TECHNICAL FIELD

The present invention relates to nonaqueous electrolytes and to secondary cells in which such nonaqueous electrolytes are used.

### BACKGROUND ART

Ionic compounds generally exist in the form of crystals composed of positively charged cations and negatively charged anions which pull electrostatically on each other. Such ionic compounds dissolve in water and various other liquids to form liquids that conduct electricity, i.e., electrolyte solutions.

Some ionic compounds maintain a liquid state at room temperature and do not solidify even at very low temperatures. Such ionic compounds which maintain a liquid state at room temperature or below are referred to in particular as "room-temperature fused salts" or "ionic liquids." To minimize electrostatic interactions between the cations and anions which make up the ionic liquid, either or both are molecular ions of a substantial size. Moreover, to minimize the charge and electrostatic interactions, either or both are monovalent.

Active research efforts are being made to employ such ionic liquids as electrolytes in batteries and other applications. However, because ionic liquids generally have a high hydroscopic property and are difficult to handle in air, their use has remained limited.

In light of these circumstances, the 1-ethyl-3-methylimidazolium tetrafluoroborate reported by Wilkes et al. in 1992 is a remarkable ionic liquid that can be handled even in air. This new ionic liquid led to the synthesis of many ionic liquids which are combinations of numerous alkylimidazolium cations having different side chains with various anions.

Since the above report was published, efforts to use ionic liquids as the electrolyte in nonaqueous electrolyte secondary cells have gradually begun to be made. For example, JP-A 11-260400 describes nonaqueous electrolyte secondary cells which use a nonaqueous electrolyte composed of a room-temperature fused salt (ionic liquid) and a carbonate compound.

However, the room-temperature fused salts used in the above publication are primarily imidazolium-based salts. Because these have relatively high solidification points, batteries obtained using such salts have less than satisfactory low temperature characteristics.

In addition, efforts have been made to suppress the reductive decomposition of room-temperature fused salts by adding a carbonate. However, because the salt itself does not have a very broad potential window, it is readily subject to reductive decomposition during charging and discharging of the battery and thus lacks a performance adequate for practical use.

It is therefore one object of the invention is to provide nonaqueous electrolytes which contain an ionic liquid and provide secondary cells having excellent low-temperature characteristics and stability. Another object of the invention is to provide nonaqueous electrolyte secondary cells which contain such nonaqueous electrolytes.

### DISCLOSURE OF THE INVENTION

The inventors have conducted extensive investigations in order to achieve the above objects. As a result they have found that quaternary ammonium salts and quaternary phosphonium salts bearing at least one alkoxyalkyl group as a substituent have the properties of ionic liquids. Moreover, the inventors have found that because these ionic liquids have a low melting point and a broad potential window, they are not readily subject to reductive decomposition during charging and discharging of the battery, and are thus excellent nonaqueous electrolytes. These discoveries led ultimately to the present invention.

Accordingly, the invention provides the following:

(1) A nonaqueous electrolyte characterized by containing an ionic liquid having general formula (1) below and a melting point not higher than 50°C

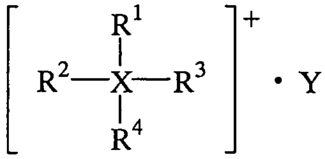

(1)

wherein $R^1$ to $R^4$ are each independently an alkyl group of 1 to 5 carbons or an alkoxyalkyl group of the formula $R'-O-(CH_2)_n-$ (R' being methyl or ethyl, and the letter n being an integer from 1 to 4), and any two from among $R^1$, $R^2$, $R^3$ and $R^4$ may together form a ring, with the proviso that at least one of $R^1$ to $R^4$ is an alkoxyalkyl group of the above formula, and wherein X is a nitrogen atom or a phosphorus atom and Y is a monovalent anion; and containing also a compound which reductively decomposes at a more noble potential than the ionic liquid, and a lithium salt.

(2) The nonaqueous electrolyte of (1) above which is characterized in that the compound is reductively decomposed at a more noble potential than the ionic liquid when a working electrode used with the electrolyte is made of a carbonaceous material or metallic lithium.

(3) The nonaqueous electrolyte of (1) or (2) above which is characterized in that the content of said compound is from 0.1 to 60 wt%.

(4) The nonaqueous electrolyte of (3) above which is characterized in that the content of said compound is 0.1 to 30 wt%.

(5) The nonaqueous electrolyte of any one of (1) to (4) above which is characterized in that the compound is one or more selected from among ethylene carbonate, propylene carbonate, vinylene carbonate, dimethyl carbonate, ethyl methyl carbonate and diethyl carbonate.

(6) The nonaqueous electrolyte of any one of (1) to (5) above which is characterized in that the ionic liquid has a melting point not higher than 25°C.

(7) The nonaqueous electrolyte of any one of (1) to (6) above which is characterized in that X is a nitrogen atom, R' is methyl, and the letter n is 2.

(8) The nonaqueous electrolyte of any one of (1) to (6) above which is characterized in that the ionic liquid has general formula (2) below

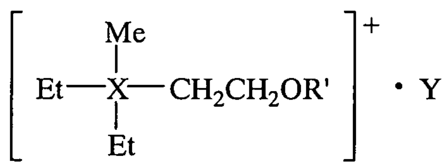

(2)

(wherein R' is methyl or ethyl, X is a nitrogen atom or a phosphorus atom, Y is a monovalent anion, Me stands for methyl and Et stands for ethyl).

(9) The nonaqueous electrolyte of any one of (1) to (8) above which is characterized in that Y is $BF_4^-$, $PF_6^-$, $(CF_3SO_2)_2N^-$, $CF_3SO_3^-$ or $CF_3CO_2^-$.

(10) The nonaqueous electrolyte of any one of (1) to (9) above which is characterized in that the lithium salt is $LiBF_4$, $LiPF_6$, $Li(CF_3SO_2)_2N$, $LiCF_3SO_3$ or $LiCF_3CO_2$.

(11) A nonaqueous electrolyte secondary cell having a positive electrode which contains a lithium-containing double oxide, a negative electrode which contains a carbonaceous material capable of inserting and extracting lithium ions or contains metallic lithium, a separator between the positive and negative electrodes, and a nonaqueous electrolyte; which secondary cell is characterized in that the nonaqueous electrolyte is a nonaqueous electrolyte according to any one of (1) to (10) above.

(12) The nonaqueous electrolyte secondary cell of (11) above which is characterized in that the separator is a porous film or porous sheet having a thickness of 20 to 50 μm and a porosity of 25 to 85%.

(13) The nonaqueous electrolyte secondary cell of (12) above which is characterized in that the porous film or porous sheet is composed primarily of cellulose.

According to this invention, because the nonaqueous electrolyte contains an ionic liquid having a low melting point and a broad potential window, and contains also a compound which reductively decomposes at a more noble potential than the ionic liquid, by using this nonaqueous electrolyte as the electrolyte in secondary cells there can be

obtained nonaqueous electrolyte secondary cells having excellent low-temperature characteristics, stability and cycle retention.

BRIEF DESCRIPTION OF THE DIAGRAMS

FIG. 1 is a graph showing potential window data for the compounds obtained in Synthesis Examples 1 to 3.
FIG. 2 is a graph showing the charge curves in the first cycle for the test cells produced in Example 1 according to the invention and in Comparative Example 1.
FIG. 3 is a graph showing the discharge curve in the first cycle for the nonaqueous electrolyte secondary cell produced in Example 9 of the invention.
FIG. 4 is a graph showing the initial charge and discharge curves for the nonaqueous electrolyte secondary cell produced in Example 13 of the invention.
FIG. 5 is a graph showing discharge curves during load characteristics tests on the nonaqueous electrolyte secondary cell produced in Example 13 of the invention.
FIG. 6 is a graph showing the percent retention of capacity up to the 100$^{th}$ cycle in the nonaqueous electrolyte secondary cell produced in Example 13 of the invention.
FIG. 7 is a graph showing discharge curves during temperature tests on the nonaqueous electrolyte secondary cell produced in Example 13 of the invention.
FIG. 8 is a graph showing discharge curves during load characteristics tests on the nonaqueous electrolyte secondary cell produced in Example 19 of the invention.

BEST MODE FOR CARRYING OUT THE INVENTION

The invention is described more fully below.

[Nonaqueous Electrolyte]

The ionic liquid used in the nonaqueous electrolyte according to the invention has general formula (1) below and has a melting point not higher than 50°C.

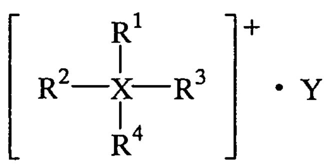

(1)

In formula (1), $R^1$ to $R^4$ are each independently an alkyl group of 1 to 5 carbons or an alkoxyalkyl group of the formula R'-O-$(CH_2)_n$- (R' being methyl or ethyl, and the letter n being an integer from 1 to 4), and any two from among $R^1$, $R^2$, $R^3$ and $R^4$ may together form a ring, with the proviso that at least one of $R^1$ to $R^4$ is an alkoxyalkyl group of the above formula. X is a nitrogen atom or a phosphorus atom, and Y is a monovalent anion.

Examples of alkyls having 1 to 5 carbons include methyl, ethyl, propyl, 2-propyl, butyl and pentyl. However, taking into account the physical properties and the electrochemical characteristics of the ionic liquid, it is preferable for at least one of groups $R^1$ to $R^4$ to be methyl, ethyl or propyl, and especially methyl or ethyl. These ethyl or propyl groups may form rings with other alkyl groups.

Examples of alkoxyalkyl groups of the formula R'-O-$(CH_2)_n$- include methoxymethyl, ethoxymethyl, methoxyethyl, ethoxyethyl, methoxypropyl, ethoxypropyl, methoxybutyl and ethoxybutyl. The letter n is an integer from 1 to 4. Taking into account the physical properties and the electrochemical characteristics of the ionic liquid, the letter n is preferably 1 or 2, and most preferably 2.

Exemplary compounds in which any two groups from among $R^1$ to $R^4$ form a ring include, when X is a nitrogen atom, quaternary ammonium salts containing an aziridine, azetidine, pyrrolidine or piperidine ring; and, when X is a phosphorus atom, quaternary phosphonium salts containing a pentamethylenephosphine (phosphorinane) ring.

Quaternary ammonium salts having as a substituent at least one methoxyethyl group, in which R' above is methyl and the letter n is 2, are preferred.

Preferred use can also be made of quaternary salts of general formula (2) below having as substituents a

methyl group, two ethyl groups and an alkoxyethyl group.

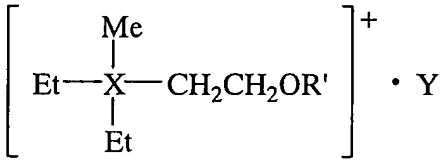

(2)

In formula (2), R' is methyl or ethyl, X is a nitrogen or phosphorus atom, and Y is a monovalent anion. Me represents a methyl group and Et represents an ethyl group.

Illustrative, non-limiting examples of the monovalent anion Y include $BF_4^-$ , $PF_6^-$, $AsF_6^-$, $SbF_6^-$, $AlCl_4^-$, $HSO_4^-$, $ClO_4^-$, $CH_3SO_3^-$, $CF_3SO_3^-$, $CF_3CO_2^-$, $(CF_3SO_2)_2N^-$, $(C_2F_5SO_2)_2N^-$, $(C_3F_7SO_2)_2N^-$, $(C_4F_9SO_2)_2N^-$, $(CF_3SO_2)(C_2F_5SO_2)N^-$, $(CF_3SO_2)(C_3F_7SO_2)N^-$, $(CF_3SO_2)(C_4F_9SO_2)N^-$, $(C_2F_5SO_2)(C_3F_7SO_2)N^-$, $(C_2F_5SO_2)(C_4F_9SO_2)N^-$, $Cl^-$, $Br^-$ and $I^-$. To ensure such properties as a good degree of dissociation and good stability in nonaqueous organic solvents, the use of $BF_4^-$, $PF_6^-$, $(CF_3SO_2)_2N^-$, $CF_3SO_3^-$ or $CF_3CO_2^-$ is preferred.

Of these anions, the use of $(CF_3SO_2)_2N^-$ is highly preferable for further reducing the viscosity of the ionic liquid and increasing its handleability. $BF_4^-$ is also highly preferable because the resulting ionic liquid has a high versatility and it is less readily affected by water than ionic liquids containing $PF_6^-$ as the anion and thus easier to handle.

Specific examples of quaternary ammonium salts and quaternary phosphonium salts highly suitable for use in the invention include compounds (3) to (11) below (wherein "Me" stands for methyl and "Et" stands for ethyl).

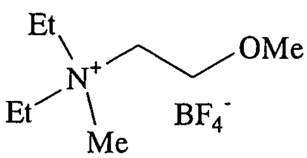

(3)

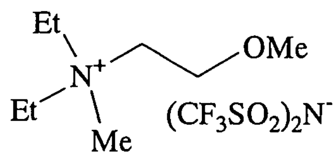

(8)

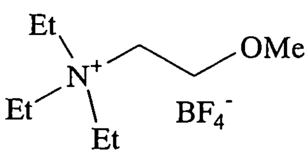

(4)

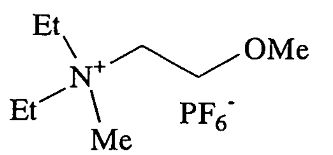

(9)

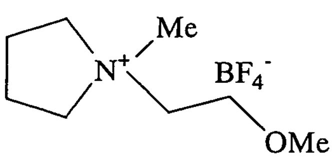

(5)

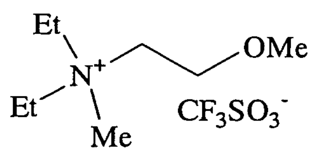

(10)

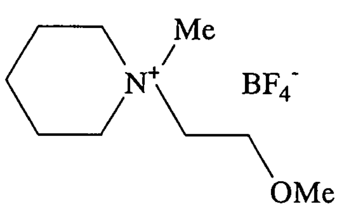

(6)

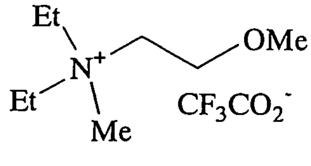

(11)

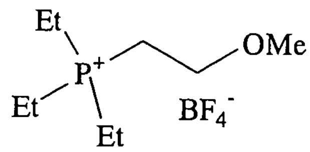

(7)

A common method for synthesizing such quaternary ammonium salts is described. First, a tertiary amine is mixed with a compound such as an alkyl halide or a dialkyl sulfate and reacted under heating, if necessary, to give a quaternary ammonium halide. In cases where a compound having a low reactivity (e.g., an alkoxyethyl halide or an alkoxymethyl halide) is used, reaction under applied pressure, such as in an autoclave, is preferred.

The resulting quaternary ammonium halide is dissolved in an aqueous solvent such as water, then reacted with a reagent that generates the required anionic species, such as tetrafluoroboric acid or tetrafluorophosphoric acid, so as to effect an anion exchange reaction, yielding the quaternary ammonium salt.

In one illustrative method for synthesizing quaternary ammonium tetrafluoroborate, a quaternary ammonium halide is dissolved in water, silver oxide is added and a salt exchange reaction is carried out to form the corresponding quaternary ammonium hydroxide. The product is then reacted with tetrafluoroboric acid, yielding the target compound. This method is effective for synthesizing high-purity quaternary ammonium tetrafluoroborates because the silver halide that arises as a result of salt exchange during formation of the quaternary ammonium hydroxide can easily be removed.

Quaternary phosphonium salts can generally be synthesized in much the same way as quaternary ammonium salts. Typically, a tertiary phosphine is mixed with a suitable compound such as an alkyl halide or a dialkyl sulfate. If necessary, the reaction is carried out under the application of heat.

As in the case of quaternary ammonium salts, quaternary phosphonium salts containing various different anions may be prepared by dissolving a quaternary phosphonium halide (a chloride, bromide or iodide) in an aqueous solvent and reacting the dissolved halide with a reagent that generates the required anionic species so as to effect an anion exchange reaction.

The above ionic liquid has a melting point not higher than 50°C, preferably not higher than 25°C, and most preferably not higher than 15°C. If the melting point is higher than 50°C, the ionic liquid will precipitate within the electrolyte at low temperatures, increasing the likelihood of a decline in the ionic conductivity. The lower the melting point, the more desirable. The melting point has no particular lower limit.

Because the ionic liquid of the invention has a lower melting point than imidazolium ion-containing ionic liquids known to the art, by using nonaqueous electrolytes containing this ionic liquid as the electrolyte in nonaqueous electrolyte secondary cells, there can be obtained secondary cells having even better low-temperature characteristics.

Also, compared with imidazolium ion-containing ionic liquids known to the art, the above ionic liquids allow the level of water present therein to be lowered or minimized. As a result, by using a nonaqueous electrolyte containing this ionic liquid as the electrolyte for a nonaqueous electrolyte secondary cell, secondary cells can be obtained which have a higher charge/discharge performance and are less subject to deterioration with repeated charging and discharging.

Furthermore, because the above ionic liquid has a broader potential window than prior-art ionic liquids containing imidazolium ions, it does not itself readily undergo reductive decomposition during charging and discharging. Preparation of the electrolyte by adding to this ionic liquid the subsequently described compound which reductively decomposes at a more noble potential than the ionic liquid enables reductive decomposition of the ionic liquid associated with charging and discharging to be more effectively suppressed. As a result, a highly stable secondary cell can be obtained which does not readily deteriorate even with repeated charging and discharging.

The nonaqueous electrolytes of the invention contain the above-described ionic liquid, a compound which reductively decomposes at a more noble potential than the ionic liquid, and a lithium salt.

The compound which is reductively decomposed at a more noble potential than the ionic liquid is not subjected to any particular limitation provided it is a compound which forms what is known as a solid electrolyte interphase (SEI) film on the active materials of secondary cells, particularly the negative electrode active material, due to the electrochemical reactions associated with charging and discharging, and which at this time reductively decomposes at a more noble potential than the ionic liquid. Illustrative examples of such compounds that may be used include cyclic and acyclic carbonates, acyclic carboxylates, cyclic and acyclic ethers, phosphates, lactone compounds, nitrile compounds, amide compounds, and mixtures thereof.

One method that may be employed to measure the potential at which reductive decomposition occurs is cyclic voltammetry which is carried out using a potentiostat and function generator in a system where the working electrode and the counter electrode are each platinum electrodes and the reference electrode is a silver/silver chloride electrode, or a system where the working electrode is made of a carbonaceous material or metallic lithium, the counter electrode is a platinum electrode and the reference electrode is a silver/silver chloride electrode. Another measurement method

that may be employed involves carrying out charge/discharge tests using a constant-current, constant-voltage power supply in a system which uses a carbonaceous material electrode as the working electrode and a metallic lithium electrode as the counter electrode and which uses, if necessary, a reference electrode such as a metallic lithium electrode.

When measuring the reduction potential of the compound that is reductively decomposed at a more noble potential than the ionic liquid, it is particularly advantageous to do so either by means of cyclic voltammetry in an actual nonaqueous electrolyte secondary cell system, that is, a system which is closely similar to a lithium ion secondary cell or lithium secondary cell and uses a carbonaceous material electrode or a metallic lithium electrode as the working electrode, a platinum electrode as the counter electrode, and a silver/silver chloride electrode as the reference electrode; or by carrying out charge/discharge tests in a system which uses a carbonaceous material electrode as the working electrode and a metallic lithium electrode as the counter electrode and which, if necessary, uses a reference electrode such as a metallic lithium electrode.

Examples of suitable cyclic carbonates include alkylene carbonates such as propylene carbonate, ethylene carbonate and butylene carbonate; and vinylene carbonates. Examples of suitable acyclic carbonates include dialkyl carbonates such as dimethyl carbonate, ethyl methyl carbonate and diethyl carbonate. Examples of suitable acyclic carboxylates include methyl acetate and methyl propionate. Examples of suitable cyclic and acyclic ethers include tetrahydrofuran, 1,3-dioxolane and 1,2-dimethoxyethane. Examples of suitable phosphates include trimethyl phosphate, triethyl phosphate, ethyldimethyl phosphate, diethylmethyl phosphate, tripropyl phosphate, tributyl phosphate, tri(trifluoromethyl) phosphate, tri(trichloromethyl) phosphate, tri(trifluoroethyl) phosphate, tri(triperfluoroethyl) phosphate, 2-ethoxy-1,3,2-dioxaphosphoran-2-one, 2-trifluoroethoxy-1,3,2-dioxaphosphoran-2-one and 2-methoxyethoxy-1,3,2-dioxaphosphoran-2-one. An example of a suitable lactone compound is $\gamma$-butyrolactone. An example of a suitable nitrile compound is acetonitrile. An example of a suitable amide compound is dimethylformamide.

Of these compounds, cyclic carbonates, acyclic carbonates, phosphates and mixtures thereof are preferred because they provide a good battery performance such as high charge/discharge characteristics and high output characteristics. The use of one or more selected from among ethylene carbonate, propylene carbonate, vinylene carbonate, dimethyl carbonate, ethyl methyl carbonate and diethyl carbonate is especially preferred. Of these, cyclic carbonates such as ethylene carbonate, propylene carbonate and vinylene carbonate are most preferable on account of their safety owing to their high flash and ignition points and their low vapor pressures.

In particular, the use of vinylene carbonate is especially desirable because it reductively decomposes at a more noble potential than the other carbonate compounds, and forms a thin, uniform SEI layer. That is, the use of vinylene carbonate enables an ionic liquid having a relatively narrow potential window to be used as the electrolyte. Moreover, even in cases where such an ionic liquid is used, the vinylene carbonate reductively decomposes and forms a SEI layer that is thin and uniform, making it possible to suppress a rise in impedance in nonaqueous electrolyte secondary cells due to SEI layer growth.

In the above-described nonaqueous electrolyte, the content of the compound which reductively decomposes at a more noble potential than the ionic liquid is preferably 0.1 to 60 wt%, more preferably 0.1 to 30 wt%, even more preferably 0.2 to 20 wt%, and most preferably 0.4 to 15 wt%, based on the overall nonaqueous electrolyte.

At a content of the above compound of less than 0.1 wt%, even when the above-described ionic liquid having a broad potential window is used, it is very likely to be difficult to efficiently suppress reductive decomposition of the ionic liquid during charging and discharging. As a result, improvements in the cycle life and stability of the secondary cell may not be achieved. On the other hand, at a content of more than 60 wt%, the nonflammable ionic liquid accounts for a lower proportion of the nonaqueous electrolyte, which may lower the safety of the secondary cell.

The lithium salt may be any known lithium salt capable of being used in nonaqueous electrolyte secondary cells. Illustrative examples include $LiBF_4$, $LiPF_6$, $LiAsF_6$, $LiClO_4$, $Li(CF_3SO_2)_2N$, $Li(CF_3SO_2)_2N$, $Li(C_2F_5SO_2)_2N$, $Li(C_3F_7SO_2)_2N$, $Li(C_4F_9SO_2)_2N$, $Li(CF_3SO_2)(C_2F_5SO_2)N$, $Li(CF_3SO_2)(C_3F_7SO_2)N$, $Li(CF_3SO_2)(C_4F_9SO_2)N$, $Li(C_2F_5SO_2)(C_3F_7SO_2)N$, $Li(C_2F_5SO_2)(C_4F_9SO_2)N$, $Li(CF_3OCF_2SO_2)_2N$, $LiCF_3SO_3$ and $LiCF_3CO_2$. To ensure such properties as versatility and good solubility and a good degree of dissociation in the ionic liquid, the use of $LiBF_4$, $LiPF_6$, $Li(CF_3SO_2)_2N$, $LiCF_3SO_3$ or $LiCF_3CO_2$ is especially preferred.

The content of lithium salt in the above electrolyte is not subject to any particular limitation, although the content is generally 0.05 to 3 mol/L, and preferably 0.1 to 2 mol/L. Too low a lithium salt concentration may result in a higher cell impedance, which make charging and discharging at a large current impossible. On the other hand, a lithium salt concentration which is too high increases the liquid viscosity, which may make battery production difficult.

Because the above-described nonaqueous electrolyte of the invention contains an ionic liquid having a broader potential window than imidazolium ion-containing ionic liquids known to the prior art and contains also a compound which reductively decomposes at a more noble potential than the ionic liquid, the ionic liquid does not readily incur reductive decomposition during charging and discharging. This enables the cycle retention and stability of nonaqueous electrolyte secondary cells in which this electrolyte is used to be improved.

Also, because the above ionic liquid has a lower melting point than imidazolium ion-containing ionic liquids

known to the prior art, a nonaqueous electrolyte having even better low-temperature characteristics can be obtained.

It is also possible for the above-described nonaqueous electrolyte of the invention to be mixed with a polymeric compound and used as a polymer electrolyte.

No particular limitation is imposed on the polymeric compound. Some preferred polymeric compounds are (a) hydroxyalkyl polysaccharide derivatives, (b) oxyalkylene branched polyvinyl alcohol derivatives, (c) polyglycidol derivatives, (d) cyano-substituted monovalent hydrocarbon group-bearing polyvinyl alcohol derivatives, and (e) thermoplastic polyurethanes.

Illustrative examples of (a) hydroxyalkyl polysaccharide derivatives include:
(1) hydroxyethyl polysaccharides prepared by reacting ethylene oxide with a naturally occurring polysaccharide such as cellulose, starch or pullulan; (2) hydroxypropyl polysaccharides prepared by reacting propylene oxide with the above naturally occurring polysaccharides; and (3) dihydroxypropyl polysaccharides prepared by reacting glycidol or 3-chloro-1,2-propanediol with the above naturally occurring polysaccharides. Hydroxyalkyl polysaccharide derivatives in which some or all of the hydroxyl groups on these hydroxyalkyl polysaccharides are capped with an ester-bonded or ether-bonded substituent are preferred.

The above hydroxyalkyl polysaccharides have a molar substitution of 2 to 30, and preferably 2 to 20. At the molar substitution of less than 2, the lithium salt dissolving ability of the hydroxyalkyl polysaccharide may become so low as to make it unsuitable for use.

Oxyalkylene branched polyvinyl alcohol derivatives (b) suitable for use as the polymeric compound include polymeric compounds which bear on the molecule polyvinyl alcohol units of general formula (12) below, which have an average degree of polymerization of at least 20, and in which some or all of the hydroxyl groups on the polyvinyl alcohol units are substituted with oxyalkylene-bearing groups having an average molar substitution of at least 0.3.

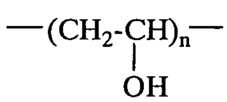

(12)

In formula (12), the letter n is preferably from 20 to 10,000.

Because this type of polymeric compound has a high oxyalkylene fraction, it has the ability to dissolve a large amount of the lithium salt. In addition, the molecule contains many oxyalkylene segments which permit the movement of ions, resulting in a high ion mobility. This type of polymeric compound is thus capable of exhibiting a high ionic conductivity. Moreover, these polymeric compounds have a high tackiness. Accordingly, they act as a binder component and are capable of firmly bonding the positive and negative electrodes.

Examples of polymeric compounds of above formula (12) include [1] polymeric compounds obtained by reacting a polyvinyl alcohol unit-containing polymeric compound with an oxirane compound such as ethylene oxide, propylene oxide or glycidol (e.g., dihydroxypropylated polyethylene vinyl alcohol, propylene oxide-modified polyvinyl alcohol); and [2] polymeric compounds obtained by reacting a polymeric compound having polyvinyl alcohol units with a polyoxyalkylene compound having terminal hydroxy-reactive substituents.

Here, the polyvinyl alcohol unit-bearing polymeric compound is a polymeric compound which has a number-average degree of polymerization of at least 20, preferably at least 30, and most preferably at least 50, which has polyvinyl alcohol units on the molecule, and in which some or all of the hydroxyl groups on the polyvinyl alcohol units are substituted with oxyalkylene-containing groups. For the sake of handleability, the upper limit in the number-average degree of polymerization in this case is preferably not more than 2,000, more preferably not more than 500, and most preferably not more than 200.

It is most preferable for the above-described polyvinyl alcohol unit-bearing polymeric compound to have a number-average degree of polymerization within the above range and to be a homopolymer in which the fraction of polyvinyl alcohol units in the molecule is at least 98 mol%. However, the polyvinyl alcohol unit-bearing polymeric compound is not limited to the above, and may be one which has a number-average degree of polymerization within the above range and which has a polyvinyl alcohol fraction of preferably at least 60 mol%, and more preferably at least 70 mol%. Illustrative examples of such compounds that may be used include polyvinyl formals in which some of the hydroxyl groups on the polyvinyl alcohol have been converted to formal, modified polyvinyl alcohols in which some of the hydroxyl groups on the polyvinyl alcohol have been converted to alkyls, poly(ethylene vinyl alcohols), partially saponified polyvinyl acetates, and other modified polyvinyl alcohols.

This polymeric compound is one in which some or all of the hydroxyl groups on the above-described polyvinyl alcohol units are substituted with oxyalkylene-containing groups having an average molar substitution of at least 0.3 (moreover, some of the hydrogen atoms on these oxyalkylene groups may be substituted with hydroxyl groups). Preferably at least 30 mol%, and most preferably at least 50 mol%, of the hydroxyl groups are substituted in this way.

The above-mentioned polyglycidol derivative (c) contains units of formula (13) (referred to hereinafter as "A units")

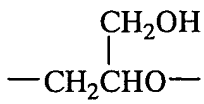

(13)

and units of formula (14) (referred to hereinafter as "B units")

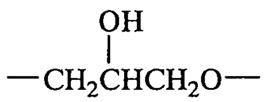

(14) .

The ends of the molecular chain are capped with specific substituents.

The polyglycidol can be prepared by polymerizing glycidol or 3-chloro-1,2-propanediol, although it is generally preferable to carry out polymerization from glycidol as the starting material and using a basic catalyst or a Lewis acid catalyst.

The total number of A and B units on the polyglycidol molecule is at least two, preferably at least six, and most preferably at least ten. There is no particular upper limit, although it is generally preferable for the total number of such units to not exceed about 10,000. The total number of these respective units may be set as appropriate based on such considerations as the flowability and viscosity required of the polyglycidol. The ratio of A units to B units in the molecule, expressed as A/B, is within a range of 1/9 to 9/1, and preferably 3/7 to 7/3. There is no regularity to the arrangement of A and B units. Any combination is possible.

The polyglycidol has a polyethylene glycol equivalent weight-average molecular weight (Mw), as determined by gel permeation chromatography (GPC), within a range of preferably 200 to 730,000, more preferably 200 to 100,000, and most preferably 600 to 20,000. The dispersity, defined as weight-average molecular weight divided by number-average molecular weight (Mw/Mn) is preferably 1.1 to 20, and most preferably 1.1 to 10.

These polymeric compounds (a) to (c) may be hydroxyl-capped polymer derivatives in which some or all, and preferably at least 10 mol%, of the hydroxyl groups on the molecule are capped with one or more type of monovalent substituent selected from among halogen atoms; substituted or unsubstituted monovalent hydrocarbon groups having 1 to 10 carbons, $R^5CO$- groups (wherein $R^5$ is a substituted or unsubstituted monovalent hydrocarbon group of 1 to 10 carbons), $R^5{}_3Si$- groups (wherein $R^5$ is as defined above), amino groups, alkylamino groups and phosphorus-containing groups.

Illustrative examples of the substituted or unsubstituted monovalent hydrocarbon groups having 1 to 10 carbons include alkyl groups such as methyl, ethyl, propyl, isopropyl, t-butyl and pentyl, aryl groups such as phenyl and tolyl, aralkyl groups such as benzyl, alkenyl groups such as vinyl, and any of the foregoing in which some or all of the hydrogen atoms have been substituted with halogen atoms, cyano groups, hydroxyl groups or amino groups. Any one or combination of two or more of these types of groups may be used.

Capping the hydroxyl groups on the above polymeric compounds (a) to (c) with highly polar substituents increases the polarity (and thus the dielectric constant) of the polymer matrix, making it possible to prevent the decline in conductivity which readily arises in a low dielectric constant polymer matrix due to the recombination of dissociated cations and counter ions (anions). Moreover, when capping is done using substituents that have fire-retarding and hydrophobic properties, the polymeric compound can be imparted with desirable characteristics, such as hydrophobicity and fire retardance.

To increase the dielectric constant of above polymeric compounds (a) to (c), the oxyalkylene chain-bearing polymeric compounds (a) to (c) are reacted with a hydroxy-reactive compound so as to cap the hydroxyl groups on these polymeric compounds with highly polar substituents.

Although the highly polar substituents used for this purpose are not subject to any particular limitation, neutral substituents are preferable to ionic substituents. Exemplary substituents include substituted and unsubstituted monovalent hydrocarbon groups of 1 to 10 carbons, and $R^5CO$- groups (wherein $R^5$ is as defined above). If necessary, capping may also be carried out with other suitable substituents, such as amino groups or alkylamino groups.

To confer polymeric compounds (a) to (c) with hydrophobic properties and fire retardance, the hydroxyl groups on the above polymeric compounds may be capped with, for example, halogen atoms, $R^5{}_3Si$- groups (wherein $R^5$ is as defined above) or phosphorus-containing groups.

Examples of suitable $R^5{}_3Si$- groups include those in which $R^5$ represents the same substituted or unsubstituted monovalent hydrocarbon groups having 1 to 10 carbons, and preferably 1 to 6 carbons, as above. $R^5$ preferably stands for alkyl groups. Trialkylsilyl groups, and especially trimethylsilyl groups, are preferred.

Additional examples of suitable substituents include amino groups, alkylamino groups and phosphorus-containing groups.

The proportion of end groups capped with the above substituents is at least 10 mol%, preferably at least 50 mol%, and most preferably at least 90 mol%. It is even possible to cap substantially all the end groups with the above substituents, representing a capping ratio of about 100 mol%.

The above-mentioned cyano-substituted monovalent hydrocarbon group-bearing polyvinyl alcohol derivative (d) is preferably a polymeric compound which bears on the molecule polyvinyl alcohol units of above general formula (12), which has an average degree of polymerization of at least 20, and in which some or all of the hydroxyl groups on the polyvinyl alcohol units are substituted with cyano-substituted monovalent hydrocarbon groups.

Because this polymeric compound has relatively short side chains, the viscosity of the electrolyte can be held to a low level.

Examples of such polymeric compounds include polyvinyl alcohols in which some or all of the hydroxyl groups have been substituted with groups such as cyanoethyl, cyanobenzyl or cyanobenzoyl. Cyanoethyl-substituted polyvinyl alcohol is especially preferred because the side chains are short.

Various known methods may be used to substitute the hydroxyl groups on the polyvinyl alcohol with cyano-substituted monovalent hydrocarbon groups.

The above-mentioned thermoplastic polyurethane (e) is preferably a thermoplastic polyurethane resin prepared by reacting (A) a polyol compound, (B) a polyisocyanate compound and, if necessary, (C) a chain elongation agent.

Suitable thermoplastic polyurethane resins include not only polyurethane resins having urethane bond, but also polyurethane-urea resins having both urethane bond and urea bond.

The polyol compound serving as component A is preferably a polyether polyol, a polyester polyol, a polyester polyether polyol, a polyester polycarbonate polyol, a polycaprolactone polyol, or a mixture thereof.

The polyol compound serving as component A has a number-average molecular weight of preferably 1,000 to 5,000, and most preferably 1,500 to 3,000. A polyol compound having too small a number-average molecular weight may lower the physical properties of the resulting thermoplastic polyurethane resin film, such as the heat resistance and tensile elongation percentage. On the other hand, too large a number-average molecular weight increases the viscosity during synthesis, which may lower the production stability of the thermoplastic polyurethane resin being prepared. The number-average molecular weights used here in connection with polyol compounds are calculated based on the hydroxyl values measured in accordance with JIS K1577.

Illustrative examples of the polyisocyanate compound serving as component B include aromatic diisocyanates such as tolylene diisocyanate, 4,4'-diphenylmethane diisocyanate, p-phenylene diisocyanate, 1,5-naphthylene diisocyanate and xylylene diisocyanate; and aliphatic or alicyclic diisocyanates such as hexamethylene diisocyanate, isophorone diisocyanate, 4,4'-dicyclohexylmethane diisocyanate and hydrogenated xylylene diisocyanate.

The chain elongation agent serving as component C is preferably a low-molecular-weight compound having a molecular weight of not more than 300 and bearing two active hydrogen atoms capable of reacting with isocyanate groups.

Various known compounds may be used as such low-molecular-weight compounds. Illustrative examples include aliphatic diols such as ethylene glycol, propylene glycol and 1,3-propanediol; aromatic or alicyclic diols such as 1,4-bis(β-hydroxyethoxy)benzene, 1,4-cyclohexanediol and bis(β-hydroxyethyl) terephthalate; diamines such as hydrazine, ethylenediamine, hexamethylenediamine and xylylenediamine; and amino alcohols such as adipoyl hydrazide. Any one or combinations of two or more of these may be used.

The thermoplastic polyurethane resin typically includes 5 to 200 parts by weight, and preferably 20 to 100 parts by weight, of the polyisocyanate compound serving as component B and 1 to 200 parts by weight, and preferably 5 to 100 parts by weight, of the chain elongation agent serving as component C per 100 parts by weight of the polyol compound serving as component A.

As noted above, polymer electrolytes obtained by mixing the nonaqueous electrolyte of the invention with a polymeric compound contain the above-described ionic liquid and a compound which reductively decomposes at a potential more noble than the ionic liquid. Accordingly, there can be obtained nonaqueous electrolyte secondary cells which undergo little cycle deterioration and have an excellent stability, and which are also endowed with excellent low-temperature characteristics.

Moreover, because the nonaqueous electrolyte contains the above-described polymeric compound, it can exhibit a high ionic conductivity, in addition to which it can act as a binder component and is thus fully capable of firmly bonding together the positive and negative electrodes.

Also, by adding to the above-described nonaqueous electrolyte of the invention a compound having a reactive double bond on the molecule, then reacting and curing the composition, the resulting product can be used as a polymer

electrolyte. In this invention, curing is a concept which encompasses also gelation.

That is, in cases where the nonaqueous electrolyte obtained by curing or gelating the above composition is formed into a thin film and used as the electrolyte in a secondary cell, to increase the physical strength (e.g., shape retention), a compound having a reactive double bond on the molecule is added and the compound is reacted to form a polymer. This increases the ability of the electrolyte to retain its shape.

It is particularly desirable for the compound bearing a reactive double bond on the molecule to have two or more reactive double bonds, because the reaction of this compound forms a three-dimensional network structure, making it possible to increase even further the shape retaining ability of the electrolyte.

When the nonaqueous electrolyte of the invention includes not only the above-mentioned compound having at least two reactive double bonds, but also the above-described polymeric compound, there can be obtained an electrolyte having a semi-interpenetrating polymer network (semi-IPN) structure in which the molecular chains of the polymeric compound are intertwined with the three-dimensional network structure of the polymer formed by crosslinkage of the reactive double bond-bearing compounds. The shape retention and strength of the electrolyte can thus be further increased, and its adhesive properties and ion conductivity also enhanced.

The compound having a reactive double bond on the molecule is not subject to any particular limitation. Illustrative examples include acrylates and methacrylates such as glycidyl methacrylate, glycidyl acrylate, methoxydiethylene glycol methacrylate, methoxytriethylene glycol methacrylate and methoxypolyethylene glycol methacrylate (average molecular weight, 200 to 1,200); and other compounds having one acrylic acid group or methacrylic acid group on the molecule, such as methacryloyl isocyanate, 2-hydroxymethylmethacrylic acid and N,N-dimethylaminoethylmethacrylic acid.

In cases where a semi-IPN structure is formed using such a compound having a single reactive double bond and the polymeric compound described above, it is necessary to add also a compound having at least two reactive double bonds on the molecule.

Preferred examples of the compound having two or more reactive double bonds on the molecule include divinylbenzene, divinylsulfone, allyl methacrylate, ethylene glycol dimethacrylate, diethylene glycol dimethacrylate, triethylene glycol dimethacrylate, polyethylene glycol dimethacrylate (average molecular weight, 200 to 1,000), 1,3-butylene glycol dimethacrylate, 1,6-hexanediol dimethacrylate, neopentyl glycol dimethacrylate, polypropylene glycol dimethacrylate (average molecular weight, 400), 2-hydroxy-1,3-dimethacryloxypropane, 2,2-bis[4-(methacryloxyethoxy)phenyl]propane, 2,2-bis[4-(methacryloxyethoxy-diethoxy)phenyl]propane, 2,2-bis[4-(methacryloxyethoxy-polyethoxy)phenyl]propane, ethylene glycol diacrylate, diethylene glycol diacrylate, triethylene glycol diacrylate, polyethylene glycol diacrylate (average molecular weight, 200 to 1,000), 1,3-butylene glycol diacrylate, 1,6-hexanediol diacrylate, neopentyl glycol diacrylate, polypropylene glycol diacrylate (average molecular weight, 400), 2-hydroxy-1,3-diacryloxypropane, 2,2-bis[4-(acryloxyethoxy)phenyl]propane, 2,2-bis[4-(acryloxyethoxy-diethoxy)phenyl]propane, 2,2-bis[4-(acryloxyethoxy-polyethoxy)phenyl]propane, trimethylolpropane triacrylate, trimethylolpropane trimethacrylate, tetramethylolmethane triacrylate, tetramethylolmethane tetraacrylate, water-soluble urethane diacrylate, water-soluble urethane dimethacrylate, tricyclodecane dimethanol acrylate, hydrogenated dicyclopentadiene diacrylate, polyester diacrylate and polyester dimethacrylate.

Of the aforementioned reactive double bond-bearing compounds, especially preferred reactive monomers include the polyoxyalkylene component-bearing diesters of general formula (15) below. The use of such a diester in combination with a polyoxyalkylene component-bearing monoester of general formula (16) below and a triester is recommended.

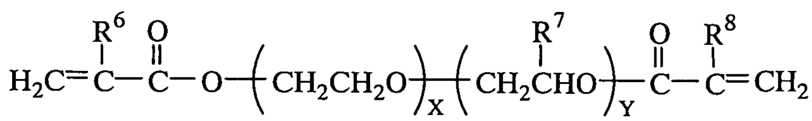

(15)

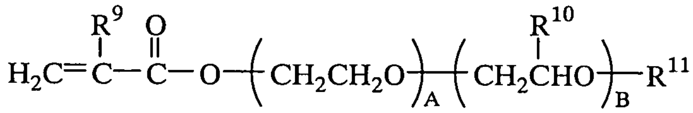

(16)

In formula (15), $R^6$ to $R^8$ are each independently a hydrogen atom or an alkyl group having 1 to 6 carbons, and preferably 1 to 4 carbons, such as methyl, ethyl, n-propyl, i-propyl, n-butyl, i-butyl, s-butyl or t-butyl; and X and Y satisfy the condition $X \geq 1$ and $Y \geq 0$ or the condition $X \geq 0$ and $Y \geq 1$. $R^6$ to $R^8$ are preferably methyl, ethyl, n-propyl, i-propyl, n-butyl, i-butyl, s-butyl or t-butyl.

In formula (16), $R^9$ to $R^{11}$ are each independently a hydrogen atom or an alkyl group having 1 to 6 carbons, and preferably 1 to 4 carbons, such as methyl, ethyl, n-propyl, i-propyl, n-butyl, i-butyl, s-butyl or t-butyl; and A and B satisfy the condition $A \geq 1$ and $B \geq 0$ or the condition $A \geq 0$ and $B \geq 1$. $R^9$ to $R^{11}$ are preferably methyl, ethyl, n-propyl, i-propyl, n-butyl, i-butyl, s-butyl or t-butyl.

A preferred example of the compound of above formula (15) is one in which X is 9, Y is 0, and both $R^6$ and $R^8$ are $CH_3$. A preferred example of the compound of above formula (16) is one in which A is 2 or 9, B is 0, and both $R^9$ and $R^{11}$ are $CH_3$.

The triester is preferably trimethylolpropane trimethacrylate.

The above-described polyoxyalkylene component-bearing diester and polyoxyalkylene component-bearing monoester are exposed, in a mixture together with the above-described ionic liquid, the compound which reductively decomposes at a more noble potential than the ionic liquid, the lithium salt and the polymeric compound, to a suitable form of radiation (e.g., UV light, electron beams, x-rays, gamma rays, microwaves, radio-frequency radiation). Alternatively, the mixture is heated to form a semi-IPN-type three-dimensional crosslinked network structure.

The relative proportions of the above-described polyoxyalkylene component-bearing diester, the polyoxyalkylene component-bearing monoester and the triester are set as appropriate for the length of the polyoxyalkylene components and are not subject to any particular limitation. However, a diester/monoester molar ratio of 0.1 to 2, and especially 0.3 to 1.5, and a diester/triester molar ratio of 2 to 15, and especially 3 to 10, are preferred to enhance the strength of the electrolyte.

As explained above, because the polymer electrolyte obtained by curing (gelating) a composition containing the nonaqueous electrolyte of the invention and a reactive double bond-containing compound includes the above-mentioned ionic liquid and a compound which reductively decomposes at a more noble potential than the ionic liquid, in addition to the above-mentioned properties such as low-temperature characteristics, cyclability, ionic isoelectricity and tackiness, the polymer electrolyte also has a high shape-retaining ability.

In particular, because a compound with at least two reactive double bonds is used as the compound having a reactive double bond on the molecule and because the electrolyte obtained by curing a composition which contains also the above-described polymeric compound has a semi-IPN type three-dimensional crosslinked network structure, the shape retaining ability and strength of the electrolyte can be increased all the more, as can also its adhesive properties and ionic conductivity.

[Secondary Cell]

The secondary cell according to this invention is a nonaqueous electrolyte secondary cell having a positive electrode which contains a lithium-containing double oxide, a negative electrode containing a carbonaceous material capable of inserting and extracting of lithium ions or containing metallic lithium, a separator between the positive and negative electrodes, and a nonaqueous electrolyte which is the above-described nonaqueous electrolyte or a polymeric electrolyte.

The positive electrode may be one that is produced by coating both the front and the back side or just one side of a positive electrode current collector with a positive electrode binder composition composed primarily of a binder polymer and a positive electrode active material.

Alternatively, a positive electrode binder composition composed primarily of a binder polymer and a positive electrode active material may be melted and blended, then extruded as a film to form a positive electrode.

The binder polymer may be any polymer capable of being used in the applications of concern here. For example, use may be made of (I) a thermoplastic resin having a swelling ratio, as defined by the formula below, in a range of 150 to 800%, (II) a fluoropolymer material, or a combination of two or more polymers of types (I) and (II).

The above thermoplastic resin (I) has a swelling ratio, as determined from the formula indicated below, within a range of 150 to 800%, preferably 250 to 500%, and most preferably 250 to 400%.

$$\text{Swelling ratio (\%)} = \frac{\text{Weight in grams of swollen thermoplastic resin after 24 hours immersion in electrolyte solution at 20°C (g)}}{\text{Weight in grams of thermoplastic resin before immersion in electrolyte solution at 20°C (g)}} \times 100,$$

A thermoplastic resin containing units of general formula (17) below

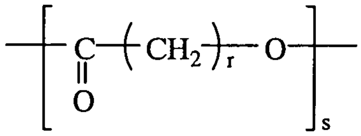

(17) ,

wherein the letter r is 3 to 5 and the letter s is an integer ≥5, may be used as the binder polymer of formula (I) above.

Preferred examples of fluoropolymer materials (II) that may be used as the binder polymer include polyvinylidene fluoride (PVDF), vinylidene fluoride-hexafluoropropylene copolymers (P(VDF-HFP)) and vinylidene fluoride-chlorotrifluoroethylene copolymers (P(VDF-CTFE)). Of these, fluoropolymers having a vinylidene fluoride content of at least 50 wt%, and especially at least 70 wt%, are preferred. The upper limit in the vinylidene fluoride content of the fluoropolymer is about 97 wt%.

The weight-average molecular weight of the fluoropolymer is not subject to any particular limitation, although the weight-average molecular weight is preferably 500,000 to 2,000,000, and most preferably 500,000 to 1,500,000. Too low a weight-average molecular weight may result in an excessive decline in physical strength.

The positive electrode current collector may be made of a suitable material such as stainless steel, aluminum, titanium, tantalum or nickel. Of these, aluminum foil or aluminum oxide foil is especially preferred both in terms of performance and cost. This current collector may be used in any of various forms, including foil, expanded metal, sheet, foam, wool, or a three-dimensional structure such as a net.

In this invention, lithium ion-containing chalcogen compounds (lithium-containing double oxides) may be used as the above positive electrode active material.

Specific examples of such lithium ion-containing chalcogen compounds (lithium-containing double oxides) include $LiCoO_2$, $LiMnO_2$, $LiMn_2O_4$, $LiMo_2O_4$, $LiV_3O_8$, $LiNiO_2$ and $Li_xNi_yM_{1-y}O_2$ (wherein M is one or more metal element selected from among cobalt, manganese, titanium, chromium, vanadium, aluminum, tin, lead and zinc; $0.05 \le x \le 1.10$; and $0.5 \le y \le 1.0$).

In addition to the binder resin and the positive electrode active material described above, if necessary, the binder composition for the positive electrode may include also an electrically conductive material. Illustrative examples of the conductive material include carbon black, Ketjenblack, acetylene black, carbon whiskers, carbon fibers, natural graphite and artificial graphite.

The positive electrode binder composition typically includes 1,000 to 5,000 parts by weight, and preferably 1,200 to 3,500 parts by weight, of the positive electrode active material and 20 to 500 parts by weight, and preferably 50 to 400 parts by weight, of the conductive material per 100 parts by weight of the binder resin.

The negative electrode may be one that is produced by coating both the front and back sides or just one side of a negative electrode current collector with a negative electrode binder composition composed primarily of a binder polymer and a negative electrode active material. The same binder polymer may be used as in the positive electrode.

Alternatively, the negative electrode binder composition composed primarily of a binder polymer and a negative electrode active material may be melted and blended, then extruded as a film to form a negative electrode.

The negative electrode current collector may be made of a suitable material such as copper, stainless steel, titanium or nickel. Of these, copper foil or a metal foil whose surface is covered with a copper plating film is especially preferred both in terms of performance and cost. The current collector used may be in any of various forms, including foil, expanded metal, sheet, foam, wool, or a three-dimensional structure such as a net.

A carbonaceous material which reversibly inserts and extracts lithium ions is used as the negative electrode active material.

Carbonaceous materials that may be used for this purpose include non-graphitizable carbonaceous materials and graphite materials. Specific examples include pyrolytic carbons, cokes (e.g., pitch coke, needle coke, petroleum coke), graphites, glassy carbons, fired organic polymeric materials (materials such as phenolic resins or furan resins that have been carbonized by firing at a suitable temperature), carbon fibers, and activated carbon.

If necessary, the binder composition for the negative electrode may include also a conductive material. Examples of conductive materials suitable for this purpose include those mentioned above for the positive electrode binder composition.

The negative electrode binder composition typically includes 500 to 1,700 parts by weight, and preferably 700 to 1,300 parts by weight, of the negative electrode active material and 0 to 70 parts by weight, and preferably 0 to 40 parts by weight, of the conductive material per 100 parts by weight of the binder polymer.

The above-described negative electrode binder compositions and positive electrode binder compositions generally are used in the form of a paste after the addition of a dispersing medium. Suitable dispersing media include polar

solvents such as N-methyl-2-pyrrolidone (NMP), dimethylformamide, dimethylacetamide and dimethylsulfamide. The dispersing medium is typically added in an amount of about 30 to 300 parts by weight per 100 parts by weight of the positive electrode or negative electrode binder composition.

No particular limitation is imposed on the method of shaping the positive and negative electrodes as thin films, although it is preferable to apply the composition by a suitable means such as roller coating with an applicator roll, screen coating, doctor blade coating, spin coating or bar coating so as to form an active material layer having a uniform thickness when dry of 10 to 200 μm, and especially 15 to 100 μm.

Illustrative, non-limiting, examples of the separator between the positive and negative electrodes include polyethylene nonwoven fabric, polypropylene nonwoven fabric, polyester nonwoven fabric, polytetrafluoroethylene porous film, kraft paper, sheet laid from a blend of rayon fibers and sisal fibers, manila hemp sheet, glass fiber sheet, cellulose-based electrolytic paper, paper made from rayon fibers, paper made from a blend of cellulose and glass fibers, and combinations thereof in the form of multilayer sheets.

The separator is not subject to any particular limitation in structure, and may have a single-layer structure or a multilayer structure composed of a plurality of laminated films or sheets.

The separator is preferably in the form of a porous film or porous sheet having a thickness of 20 to 50 μm and a porosity of 25 to 85%. At a thickness of less than 20 μm, internal shorting of the cell may arise more frequently, and at a thickness of more than 50 μm, the discharge load characteristics of the cell may be compromised. Accordingly, it is desirable for the separator to have a thickness of 25 to 40 μm, and especially 25 to 35 μm.

At a porosity of less than 25%, the permeability of the separator to the electrolyte solution may worsen. Moreover, the amount of electrolyte solution per unit volume decreases, lowering the ionic conductivity and possibly compromising the cell characteristics. A porosity greater than 85% does enhance the large current discharge characteristics of the cell, but such a separator has a lower physical strength and may thus have a poor handleability during assembly operations, in addition to which the cell tends to have a higher incidence of internal shorting. Accordingly, it is desirable for the separator to have a porosity of 25 to 85%, preferably 50 to 80%, and most preferably 65 to 75%. By using a separator of the above thickness and porosity, there can be obtained nonaqueous electrolyte secondary cells having excellent large current discharge characteristics.

The material making up such a porous film or porous sheet is not subject to any particular limitation. That is, separators made of any of the various above-described materials may be used. However, it is preferable to use a porous film or porous sheet composed primarily of cellulose. "Composed primarily of cellulose" signifies here that cellulose accounts for at least about 95 wt% of the separator when dry (i.e., in a dehydrated state).

By using such a porous film or sheet composed primarily of cellulose, the charging characteristics, rate capability, safety and manufacturability of the nonaqueous electrolyte secondary cells can be enhanced, in addition to which the overcharge characteristics can be increased.

No particular limitation is imposed here on the cellulose-containing porous film or sheet. However, the use of paper formed from cellulose fibers by a papermaking process is preferable from the standpoint of production costs, lyophilicity with respect to the electrolyte solution, and cell characteristics. Paper obtained by beating cellulose fibers can also be used.

Because the above-described cellulose has a heat resistance of at least 200°C and thus a better thermal stability than polyolefin resins, it can enhance the thermal stability of the cell. As a result, the danger of abnormal overheating such as from internal shorting due to thermal shrinkage of the separator can be avoided.

Moreover, when a separator composed primarily of cellulose is used, hydroxyl groups on the cellulose molecules strongly interact with the highly polar electrolyte molecules, giving the separator a better lyophilicity than that of a polyolefin separator. As a result, the electrolyte solution has a high rate of penetration, increasing the efficiency of work during cell assembly and preventing the deterioration of cell performance from the presence of areas of the separator not penetrated by the electrolyte solution.

In addition, because cellulose fibers have a large surface area, the surface of the fibers interacts with the electrolyte solution, increasing electrolyte retention and reducing liquid exudation. As a result, the cell can be provided with better charge-discharge characteristics, high-temperature stability and safety.

The nonaqueous electrolyte secondary cell of the invention is assembled by stacking, fan-folding, winding or forming into a laminated or coin-like shape a cell assembly composed of the separator disposed between the positive and negative electrodes, and placing the cell assembly within a battery housing such as a battery can or a laminate pack. The battery housing is mechanically sealed if it is a can or heat-sealed if it is a laminate pack. In constructing the cell, the separator is disposed between the positive electrode and the negative electrode, and the resulting cell assembly is placed within the battery housing. The cell assembly is then filled with a nonaqueous electrolyte solution. If a compound having reactive double bonds is used as the nonaqueous electrolyte, the electrolyte composition is filled into the cell assembly so that it fully penetrates the gap between the electrodes and the gaps between the separator and the electrodes, then is reacted to effect curing.

The resulting nonaqueous electrolyte secondary cell of the invention can be operated at a high capacity and

a high current without compromising such outstanding characteristics as its charge-discharge efficiency, energy density, power density and life. Moreover, the cell has a broad service temperature range.

The nonaqueous electrolyte secondary cell of the invention lends itself well to use in a variety of applications, including main power supplies for portable electronic equipment such as video cameras, notebook computers, cell phones and PHS® ("personal handyphone system") devices, uninterruptible power supplies for equipment such as personal computers -- including use as memory backup power supplies, in electric cars and hybrid cars, and together with solar cells as energy storage systems for solar power generation.

EXAMPLE

The following Synthesis Examples, Examples of the Invention and Comparative Examples are provided to illustrate the invention and do not in any way limit the invention.

In Examples 1 to 8 and Comparative Examples 1 and 2, lithium metal was used as the counter electrode in order to examine the performance on the negative electrode side of the electrolyte solution of the invention. Although it is conventional for the carbonaceous electrode to be regarded as the positive electrode, in this case because it receives lithium ions and is reduced during discharge, it is referred to herein as the negative electrode and electrochemical activity in the reducing direction is referred to as "charging."

Synthesis Example 1

Synthesis of Compound (8)

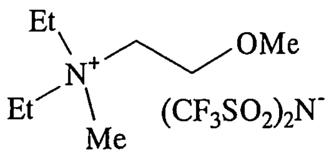

(8)

A solution prepared by mixing together 100 ml of diethylamine (Kanto Chemical Co., Inc.) and 85 ml of 2-methoxyethyl chloride (Kanto Chemical Co., Inc.) was placed in an autoclave and reacted at 100°C for 24 hours. The internal pressure during the reaction was 0.127 MPa (1.3 kgf/$cm^2$). This yielded a mixture of deposited crystals and reaction solution to which was added, following the 24 hours of reaction, 200 ml of an aqueous solution containing 56 g of dissolved potassium hydroxide (Katayama Chemical Industries Co., Ltd.). Each of the two divided organic phases that formed as a result was separated off with a separatory funnel and subjected twice to extraction with 100 ml of methylene chloride (Wako Pure Chemical Industries, Ltd.). The separated organic phases were then combined and washed with a saturated saline solution, following which potassium carbonate (Wako Pure Chemical Industries, Ltd.) was added to remove water and vacuum filtration was carried out. The solvent in the resulting organic phase was distilled off in a rotary evaporator, after which the residue was subjected to normal-pressure distillation, yielding 18.9 g of a fraction having a boiling point close to 135°C. This was identified by a $^1$H-NMR spectrum as 2-methoxyethyldiethylamine.

Next, 8.24 g of the 2-methoxyethyldiethylamine was dissolved in 10 ml of tetrahydrofuran (Wako Pure Chemical Industries, Ltd.), then 4.0 ml of methyl iodide (Wako Pure Chemical Industries, Ltd.) was added under ice cooling. After 30 minutes, the mixture was removed from the ice bath and stirred overnight at room temperature. The solvent in this reaction solution was subsequently driven off by vacuum distillation, and the resulting solids were recrystallized from an ethanol (Wako Pure Chemical Industries, Ltd.) - tetrahydrofuran system, yielding 16 g of 2-methoxyethyldiethylmethylammonium iodide.

Next, 10.0 g of the 2-methoxyethyldiethylmethylammonium iodide was dissolved in 50 mL of acetonitrile (Kanto Chemical Co. Inc.), after which 9.5 g of lithium bis(trifluoromethanesulfonyl)imide (produced by Kishida Chemical Co., Ltd.) was added and completely dissolved therein, then the resulting solution was stirred for 15 minutes.

The acetonitrile was removed by vacuum distillation, then water was added to the residue, causing the organic phase to separate into two. The organic phase was then separated off and washed five times with water to remove impurities.

The washed organic phase was subsequently placed under reduced pressure with a vacuum pump and the water was thoroughly driven off, yielding 6.8 g of compound (8), which was liquid at room temperature.

Synthesis Example 2

Synthesis of Compound (3)

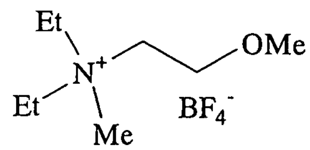

(3)

First, 8.24 g of the 2-methoxyethyldiethylamine obtained as in Synthesis Example 1 was dissolved in 10 ml of tetrahydrofuran (Wako Pure Chemical Industries, Ltd.), following which 4.0 ml of methyl iodide (Wako Pure Chemical Industries, Ltd.) was added under ice cooling. After 30 minutes, the mixture was removed from the ice bath and stirred overnight at room temperature. The solvent in the resulting reaction mixture was then driven off by vacuum distillation, and the resulting solid matter was recrystallized from an ethanol (Wako Pure Chemical Industries, Ltd.) - tetrahydrofuran system, yielding 16 g of 2-methoxyethyldiethylmethylammonium iodide.

Next, 15.0 g of the 2-methoxyethyldiethylmethylammonium iodide was dissolved in 100 ml of distilled water, following which 6.37 g of silver oxide (Kanto Chemical Co. Inc.) was added and stirring was carried out for 3 hours. The reaction mixture was then vacuum filtered to remove the precipitate, following which 42% tetrafluoroboric acid (Kanto Chemical Co. Inc.) was gradually added under stirring until the reaction solution reached a pH of about 5 to 6. The reaction solution was subsequently freeze-dried, in addition to which water was thoroughly driven off using a vacuum pump, ultimately yielding 12.39 g of a compound (3) that was liquid at room temperature (25°C).

Synthesis Example 3

First, 5.74 g of lithium bis(trifluoromethanesulfonyl)imide (produced by Kishida Chemical Co., Ltd.) was added to 30 ml of a 1:1 (by volume) mixed solvent composed of chloroform and acetonitrile, and the mixture was stirred to form a suspension. Next, a solution prepared by dissolving 2.92 g of 1-ethyl-3-methylimidazolium chloride (Tokyo Kasei Kogyo Co., Ltd.) in 30 ml of a 1:1 (by volume) mixed solvent of chloroform and acetonitrile was added to the suspension, and the mixture was stirred for 80 minutes. The crystals that formed were removed by vacuum filtration, and the solvent within the filtrate was driven off with an evaporator and a vacuum pump.

Next, 4.85 g of the resulting residue was further purified by silica gel column chromatography (Wakogel C-200, produced by Wako Pure Chemical Industries, Ltd.; eluate, 1:1 (by volume) mixed solvent of chloroform and methanol), yielding 3.06 g of an imidazolium-based ionic liquid which is liquid at room temperature.

Synthesis Example 4

A reactor equipped with a stirrer, a thermometer and a condensing tube was charged with 60.20 parts by weight of a preheated and dehydrated polyethylene glycol 4000 (PEG 4000-S, made by Sanyo Chemical Industries, Ltd.) and 7.84 parts by weight of 4,4'-diphenylmethane diisocyanate. The reactor contents were stirred and mixed for 2 hours at 120°C under a stream of nitrogen, following which 1.86 parts by weight of 1,4-butanediol was added to the mixture and the reaction was similarly effected at 120°C under a stream of nitrogen. When the reaction reached the point where the reaction product became rubbery, it was stopped. The reaction product was then removed from the reactor and heated at 100°C for 12 hours. Once the isocyanate peak was confirmed to have disappeared from the infrared absorption spectrum, heating was stopped, yielding a solid polyurethane resin.

The resulting polyurethane resin had a weight-average molecular weight (Mw) of $1.05\times10^5$. Eight parts by weight of the polyurethane resin was dissolved in 92 parts by weight of N-methyl-2-pyrrolidone to give a polyurethane resin solution.

Reference Example 1

Measurement of Potential Window

Cyclic voltammetry measurements were carried out with a potentiostat and function generator at a sweep rate of 1 mV/s on each of the compounds obtained in above Synthesis Examples 1, 2 and 3, and on 1-ethyl-3-methylimidazolium tetrafluoroborate (Kanto Chemical Co. Inc.). A platinum electrode (electrode surface area, 1.76 $mm^2$) was used as the working electrode, a platinum electrode (electrode surface area, 400 $mm^2$) was used as the counter electrode, and a silver/silver chloride electrode was used as the reference electrode. The potential window data obtained as a result of the measurements are shown in FIG. 1.

Example 1 [Test Cell 1]

<Fabrication of Carbonaceous Negative Electrode>

Ninety-two parts by weight of mesocarbon microbeads (MCMB6-28, produced by Osaka Gas Chemicals Co., Ltd.) as the carbonaceous negative electrode active material, 80 parts by weight of a solution of 10 parts by weight of polyvinylidene fluoride dissolved in 90 parts by weight of N-methyl-2-pyrrolidone, and 40 parts by weight of N-methyl-2-pyrrolidone were stirred and mixed to give a paste-like carbonaceous negative electrode-forming composition. The composition was coated onto copper foil with a doctor blade, dried at 80°C for 2 hours, then roll pressed to an electrode thickness of 30 μm so as to form a carbonaceous negative electrode.

<Preparation of Electrolyte Solution>

An electrolyte solution was prepared by first dissolving 4 parts by weight of lithium bis(trifluoromethanesulfonyl)imide in 96 parts by weight of the ionic liquid obtained in Synthesis Example 1, then adding thereto 10 parts by weight of vinylene carbonate.

<Production of Test Cell>

The carbonaceous negative electrode obtained as described above was cut to a diameter of 12 mm and a separator (E25MMS, a polyolefin porous film made by Tonen Tapyrus Co., Ltd.) was cut to a diameter of 13 mm, following which both were immersed in the electrolyte solution prepared as described above and vacuum impregnated with the solution for 30 minutes. The carbonaceous negative electrode impregnated with the electrolyte solution and a 12 mm diameter stamped disk of lithium metal were stacked together with the electrolyte solution-impregnated separator therebetween, then hermetically sealed within an outer case to give a test cell. The thickness and porosity of the polyolefin porous film used in this example were measured and found to be 25 μm and 26.6%, respectively.

The thickness of the separator was measured here using a 1/10,000 thickness gauge. The porosity was determined by cutting the separator into a 3 cm diameter disk, measuring the volume and weight of the disk in air, and using the following formula to calculate the porosity. The thickness and porosity of the separator in each of the following examples were determined in the same way.

$$\text{Porosity (\%)} = 100 \times [(\text{volume} - \text{weight})/(\text{specific gravity of resin component of separator})]/\text{volume}$$

The specific gravity of the resin component of the separator is the value obtained by the water displacement method described in JIS K 7112; that is the value calculated from the following formula using the weight of the separator in air and the weight of the separator when immersed in water.

$$\text{Specific gravity of resin component in separator} = (\text{weight of separator in air})/[(\text{weight of separator in air}) - (\text{weight of separator when immersed in water})]$$

<Charge/Discharge Test>

The test cell produced as described above was subjected to a charge/discharge test at a charge voltage of 10 mV, a discharge voltage of 1.5 V, and under a constant current at a current density of 0.03 $mA/cm^2$. The carbonaceous negative electrode had a charge capacity of 297 mAh/g and a charge/discharge efficiency in the first cycle of 83.3%.

Comparative Example 1 [Test Cell 2]

Aside from using as the electrolyte solution 4 parts by weight of lithium bis(trifluoromethanesulfonyl)imide dissolved in 96 parts by weight of the ionic liquid obtained in Synthesis Example 1, a test cell was produced in the same way as in Example 1.

<Charge/Discharge Test>

The resulting test cell was subjected to a charge/discharge test under the same conditions as in Example 1. The carbonaceous negative electrode had a capacity of 151 mAh/g, and the cell had a charge/discharge efficiency in the first cycle of 45.7%.

FIG. 2 shows the charge curves in the first cycle for the test cells produced in Example 1 and Comparative Example 1.

Comparative Example 2 [Test Cell 3]

Aside from using an electrolyte solution prepared by dissolving 4 parts by weight of lithium bis(trifluoromethanesulfonyl)imide in 96 parts by weight of the ionic liquid obtained in Synthesis Example 3 and adding thereto 10 parts by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a capacity of 243 mAh/g, and the cell had a charge/discharge efficiency in the first cycle of 68.2%.

Example 2 [Test Cell 4]

Aside from using an electrolyte solution prepared by dissolving 4 parts by weight of lithium bis(trifluoromethanesulfonyl)imide in 96 parts by weight of the ionic liquid obtained in Synthesis Example 1 and adding thereto 5 parts by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a capacity of 285 mAh/g, and the cell had a charge/discharge efficiency in the first cycle of 82.7%.

Example 3 [Test Cell 5]

Aside from using an electrolyte solution prepared by dissolving 4 parts by weight of lithium bis(trifluoromethanesulfonyl)imide in 96 parts by weight of the ionic liquid obtained in Synthesis Example 1 and adding thereto 1 part by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a capacity of 279 mAh/g, and the cell had a charge/discharge efficiency in the first cycle of 81.5%.

Example 4 [Test Cell 6]

Aside from using an electrolyte solution prepared by dissolving 4 parts by weight of lithium bis(trifluoromethanesulfonyl)imide in 96 parts by weight of the ionic liquid obtained in Synthesis Example 1 and adding thereto 10 parts by weight of ethylene carbonate, a test cell was produced in the same way as in Example 1.

<Charge/Discharge Test>

The resulting test cell was subjected to a charge/discharge test under the same conditions as in Example 1. The carbonaceous negative electrode had a charge capacity of 273 mAh/g, and the cell had a charge/discharge efficiency in the first cycle of 79.3%.

Example 5 [Test Cell 7]

Aside from using an electrolyte solution prepared by dissolving 4 parts by weight of lithium bis(trifluoromethanesulfonyl)imide in 96 parts by weight of the ionic liquid obtained in Synthesis Example 1 and adding thereto 10 parts by weight of a 1:1 (by volume) mixed solution of vinylene carbonate and diethyl carbonate, a test cell was produced in the same way as in Example 1.

<Charge/Discharge Test>

The resulting test cell was subjected to a charge/discharge test under the same conditions as in Example 1. The carbonaceous negative electrode had a capacity of 277 mAh/g, and the cell had a charge/discharge efficiency in the first cycle of 80.8%.

Example 6 [Test Cell 8]

Aside from using an electrolyte solution prepared by dissolving 17 parts by weight of lithium bis(trifluoromethanesulfonyl)imide in 83 parts by weight of the ionic liquid obtained in Synthesis Example 1 and adding thereto 10 parts by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a charge capacity of 270 mAh/g, and the cell had a charge/discharge efficiency in the first cycle of 78.2%.

Example 7 [Test Cell 9]

Aside from using an electrolyte solution prepared by dissolving 4 parts by weight of lithium tetrafluoroborate in 96 parts by weight of the ionic liquid obtained in Synthesis Example 2 and adding thereto 10 parts by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a charge capacity of 282 mAh/g, and the cell had a charge/discharge efficiency in the first cycle of 81.9%.

Example 8 [Test Cell 10]

Aside from using an electrolyte solution prepared by dissolving 4 parts by weight of lithium tetrafluoroborate in 96 parts by weight of the ionic liquid obtained in Synthesis Example 1 and adding thereto 10 parts by weight of vinylene carbonate, a test cell was produced in the same way as in Example 1.

<Charge/Discharge Test>

The resulting test cell was subjected to a charge/discharge test under the same conditions as in Example 1. The carbonaceous negative electrode had a charge capacity of 288 mAh/g, and the cell had a charge/discharge efficiency in the first cycle of 82.6%.

Table 1 below shows the capacity of the carbonaceous negative electrode and the charge/discharge efficiency in the first cycle for the cells produced in each of the above examples of the invention and the comparative examples.

Table 1

| | Capacity of carbonaceous negative electrode (mAh/g) | Charge-discharge efficiency (%) |
|---|---|---|
| Example 1 | 297 | 83.3 |
| Example 2 | 285 | 82.7 |
| Example 3 | 279 | 81.5 |
| Example 4 | 273 | 79.3 |
| Example 5 | 277 | 80.8 |
| Example 6 | 270 | 78.2 |
| Example 7 | 282 | 81.9 |
| Example 8 | 288 | 82.6 |
| Comparative Example 1 | 151 | 45.7 |
| Comparative Example 2 | 243 | 68.2 |

As is apparent in Table 1, because the test cells obtained in Examples 1 to 8 use nonaqueous electrolytes which include the ionic liquid of the invention and a compound which reductively decomposes at a more noble potential than the ionic liquid, the capacity of the carbonaceous negative electrode capacity and the charge/discharge efficiency of the cell in each of these examples are both much higher than in the test cell obtained in Comparative Example 1, which uses an electrolyte that does not include such a compound.

Moreover, the negative electrode capacity and the charge-discharge efficiency are both greatly enhanced compared also with the test cell obtained in Comparative Example 2, which uses an electrolyte that includes both an imidazolium ion-containing ionic liquid and the above type of compound.

Example 9 [Secondary Cell 1]

<Fabrication of Positive Electrode>

Ninety-two parts by weight of $LiCoO_2$ as the positive electrode active material, 3 parts by weight of Ketjenblack as the conductive material, 50 parts by weight of a solution of 10 parts by weight of polyvinylidene fluoride in 90 parts by weight of N-methyl-2-pyrrolidone, and 20 parts by weight of N-methyl-2-pyrrolidone were stirred and mixed together, giving a paste-like positive electrode composition. This positive electrode composition was applied onto aluminum foil with a doctor blade, then dried at 80°C for 2 hours and roll-pressed to an electrode thickness of 30 μm, thereby forming a positive electrode.

<Fabrication of Carbonaceous Negative Electrode>

Ninety-two parts by weight of mesophasecarbon microbeads (MCMB6-28, made by Osaka Gas Chemicals Co., Ltd.) as the carbonaceous negative electrode active material, 80 parts by weight of a solution of 10 parts by weight of polyvinylidene fluoride in 90 parts by weight of N-methyl-2-pyrrolidone, and 40 parts by weight of N-methyl-2-pyrrolidone were stirred and mixed together, giving a paste-like carbonaceous negative electrode composition. This carbonaceous negative electrode binder composition was applied onto copper foil with a doctor blade, then dried at 80°C for 2 hours and roll-pressed to an electrode thickness of 30 μm, thereby forming a carbonaceous negative electrode.

<Preparation of Electrolyte Solution>

An electrolyte solution was prepared by dissolving 4 parts by weight of lithium bis(trifluoromethanesulfonyl) imide in 96 parts by weight of the ionic liquid obtained in Synthesis Example 1, then adding thereto 10 parts by weight of vinylene carbonate.

<Production of Secondary Cell>

The positive electrode and the carbonaceous negative electrode obtained as described above were cut to

respective diameters of 11 mm and 12 mm, and a separator (E25MMS, a polyolefin porous film made by Tonen Tapyrus Co., Ltd.) was cut to a diameter of 13 mm. All three were immersed in the electrolyte solution prepared as described above, and impregnated with the solution for 30 minutes under a vacuum. The positive electrode and carbonaceous negative electrode impregnated with the electrolyte solution were stacked together, with the electrolyte solution-impregnated separator therebetween, then hermetically sealed within an outer case, thereby giving a secondary cell.

<Charge/Discharge Test>

The secondary cell produced as described above was subjected to a charge/discharge test at a charge voltage of 4.2 V, a discharge voltage of 2.7 V, and under a constant current at a current density of 0.03 $mA/cm^2$. The cell was found to have a capacity of 0.723 mAh and a charge-discharge efficiency in the first cycle of 76.5%. A 20-cycle charge-discharge test was subsequently carried out under the same conditions, whereupon the percent retention of the discharge capacity in the $20^{th}$ cycle, defined as the ratio of the discharge capacity in the $20^{th}$ cycle to the discharge capacity in the first cycle, was 97.2%. FIG. 3 shows the discharge curve in the first cycle.

Example 10 [Secondary Cell 2]

<Production of Secondary Cell>

Aside from using an electrolyte solution prepared by dissolving 4 parts by weight of lithium tetrafluoroborate in 96 parts by weight of the ionic liquid obtained in Synthesis Example 2 and adding thereto 10 parts by weight of vinylene carbonate, a secondary cell was produced in the same way as in Example 9.

<Charge/Discharge Test>

The resulting secondary cell was subjected to a charge/discharge test under the same conditions as in Example 9. The cell capacity was 0.714 mAh and the charge-discharge efficiency in the first cycle was 75.9%. A 20-cycle charge/discharge test was then carried out in the same way as in Example 9, whereupon the percent retention of discharge capacity in the $20^{th}$ cycle was found to be 96.8%.

Example 11 [Secondary Cell 3]

<Fabrication of Polyurethane Resin Film>

The polyurethane resin solution obtained in Synthesis Example 3 was applied with a doctor blade to a dry film thickness of 30 μm, then vacuum dried at 120°C for 2 hours to form a polyurethane resin film.

<Preparation of Electrolyte Solution>

An electrolyte solution was prepared by dissolving 4 parts by weight of lithium bis(trifluoromethanesulfonyl) imide in 96 parts by weight of the ionic liquid obtained in Synthesis Example 1, then adding 10 parts by weight of vinylene carbonate.

<Production of Secondary Cell>

A positive electrode and a carbonaceous negative electrode fabricated in the same way as in Example 9 were cut to respective diameters of 11 mm and 12 mm, immersed in the electrolyte solution prepared as described above, and impregnated with the solution for 30 minutes under a reduced pressure. In addition, the above-described polyurethane resin film was cut to a diameter of 13 mm and immersed for 24 hours in the electrolyte solution prepared as described above to impregnate it with the electrolyte solution. The positive electrode and carbonaceous negative electrode impregnated with the electrolyte solution were stacked together, with the electrolyte solution-impregnated polyurethane resin film therebetween, then hermetically sealed within an outer case, thereby giving a secondary cell.

<Charge/Discharge Test>

The resulting secondary cell was subjected to a charge/discharge test under the same conditions as in Example 9. The cell capacity was 0.708 mAh and the charge-discharge efficiency in the first cycle was 75.5%. A 20-cycle charge/discharge test was then carried out in the same way as in Example 9, whereupon the percent retention of

discharge capacity in the 20th cycle was found to be 96.1%.

Example 12 [Secondary Cell 4]

<Preparation of Electrolyte Composition Solution>

The following dehydration-treated components were mixed in the indicated amounts: 100 parts by weight of polyethylene glycol dimethacrylate (number of oxirene units, 9), 70.15 parts by weight of methoxypolyethylene glycol monomethacrylate (number of oxirene units, 2), and 8.41 parts by weight of trimethylolpropane trimethacrylate. To 14.5 parts by weight of the resulting mixture was added 85 parts by weight of the electrolyte solution prepared in Example 11 and 0.5 part by weight of azobisisobutyronitrile, giving an electrolyte composition.

<Production of Secondary Cell>

The positive electrode and carbonaceous negative electrode obtained in the same way as in Example 9 were cut to respective diameters of 11 mm and 12 mm, and a separator (E25MMS, a polyolefin porous film made by Tonen Tapyrus Co., Ltd.) was cut to a diameter of 13 mm. All three were immersed in the electrolyte composition solution prepared as described above and impregnated with the solution for 30 minutes under a vacuum. The positive electrode and carbonaceous negative electrode impregnated with the electrolyte composition solution were stacked together, with the electrolyte composition solution-impregnated separator therebetween. The resulting assembly was hermetically sealed within an outer case, then heated at 55°C for 2 hours and at 80°C for 0.5 hour to induce gelation, thereby giving a secondary cell.

<Charge/Discharge Test>

The resulting secondary cell was subjected to a charge/discharge test under the same conditions as in Example 9. The cell capacity was 0.706 mAh and the charge/discharge efficiency in the first cycle was 75.0%. A 20-cycle charge-discharge test was subsequently carried out under the same conditions as in Example 9, whereupon the percent retention of discharge capacity in the 20th cycle was found to be 95.3%.

The discharge capacity, charge-discharge efficiency in the first cycle and percent retention of discharge capacity for the secondary cells obtained in each of the preceding examples are given below in Table 2.

Table 2

| | Discharge capacity (mAh) | Charge/discharge efficiency | Retention of discharge capacity |
|---|---|---|---|
| Example 9 | 0.723 | 76.5 | 97.2 |
| Example 10 | 0.714 | 75.9 | 96.8 |
| Example 11 | 0.708 | 75.5 | 96.1 |
| Example 12 | 0.706 | 75.0 | 95.3 |

As is apparent in Table 2, the nonaqueous electrolyte secondary cells of Examples 9 to 12 which were produced using an electrolyte that includes the ionic liquid of the invention all exhibited excellent discharge capacities, charge-discharge efficiencies and percent retention of discharge capacity.

Example 13 [Secondary Cell 5]

<Preparation of Electrolyte Solution>

An electrolyte solution was prepared by dissolving 29 parts by weight of lithium bis(trifluoromethanesulfonyl) imide in 71 parts by weight of the ionic liquid obtained in Synthesis Example 1, then adding 10 parts by weight of vinylene carbonate.

<Production of Secondary Cell>

The positive electrode and carbonaceous negative electrode produced in the same way as in Example 9 were cut to respective dimensions, in the coated portions of the electrode, of 38x38 mm and 40x40 mm, stacked together

with an intervening cellulose separator (TF40-30, made by Nippon Kodoshi Corporation), then sandwiched and fixed in place between polypropylene sheets. This laminate was placed in a laminate pack, following which the electrolyte solution prepared as described above was poured therein and impregnated into the laminate for 1 hour under a vacuum. The laminate was then hermetically sealed in a vacuum state, giving a laminate-type nonaqueous electrolyte secondary cell. The thickness and porosity of the cellulose separator used in the invention were measured and found to be 30 μm and 73.1%, respectively.

<Charge/Discharge Test>

Initial Performance Test

Secondary cells produced as described above were subjected to a charge/discharge test at a charge voltage of 4.2 V, a discharge voltage of 2.7 V, and a current density of 0.076 mA/$cm^2$ (corresponds to 0.1 C). The discharge capacity was 11.2 mAh (capacity based on $LiCoO_2$, 132.6 mAh/g), and good operation as a lithium ion battery was confirmed. The charge and discharge curves obtained are shown in FIG. 4.

Load Characteristics Test

Secondary cells obtained as described above were subjected to a charge-discharge test under the following conditions: charge voltage, 4.2 V; discharge voltage, 2.7 V; current density while charging of 0.076 mA/$cm^2$ (corresponds to 0.1C); current densities while discharging of 0.152 mA/$cm^2$ (0.2C), 0.228 mA/$cm^2$ (0.3C), 0.380 mA/$cm^2$ (0.5C) and 0.760 mA/$cm^2$ (1.0C). The discharge capacity and the volume ratio during 0.1C discharge ((capacity at a particular discharge rate)/(capacity during 0.1C discharge) × 100) were respectively 11.1 mAh and 99.3%, 11.1 mAh and 99.1%, 10.8 mAh and 96.3%, and 6.3 mAh and 56.4%. Table 3 below shows the capacity during discharge at the various current densities, and the capacity ratio with respect to 0.1C discharge. The resulting discharge curves are shown in FIG. 5.

Table 3

| Discharge rate | Discharge capacity with respect to $LiCoO_2$ (mAh/g) | Load characteristics (vs. 0.1C) (%) |
|---|---|---|
| 0.1C | 132.6 | 100 |
| 0.2C | 131.7 | 99.3 |
| 0.3C | 131.4 | 99.1 |
| 0.5C | 127.7 | 96.3 |
| 1.0C | 74.8 | 56.4 |

Cycle Tests

Secondary cells produced as described above were subjected to a constant-current cycle test at a charge voltage of 4.2 V, a discharge voltage of 2.7 V and a current density of 0.076 mA/$cm^2$ (corresponds to 0.1C). The percent retention of the discharge capacity in the $100^{th}$ cycle, defined as the ratio of the discharge capacity in the $100^{th}$ cycle to the discharge capacity in the first cycle, was 96.9%. When a secondary cell produced in the same way was subjected to cycle tests in which only the current density during discharge was changed to a constant current of 0.380 mA/$cm^2$ (corresponds to 0.5C), the percent retention of discharge capacity in the $100^{th}$ cycle was 96.4%. FIG. 6 shows the percent retention of discharge capacity results after various numbers of cycles in cycle tests conducted at a discharge current density of 0.076 mA/$cm^2$ (corresponds to 0.1C). These results show that the cyclability of nonaqueous electrolyte secondary cells in which the nonaqueous electrolyte of the invention is used do not have a large dependence on the discharge current.

Temperature Characteristics Tests

Using secondary cells produced as described above, charging was carried out at a cut-off voltage of 4.2 V, a current density of 0.076 mA/$cm^2$ (corresponds to 0.1C) and a temperature of 25°C, and discharging was carried out at an end-of-discharge voltage of 2.7 V, a current density of 0.76 mA/$cm^2$ (corresponds to 1.0C) and at temperatures of 25°C, 45°C and 60°C. The results were a discharge capacity of 7.57 mAh during 25°C discharge, a discharge capacity of 8.28 mAh during 45°C discharge, and a discharge capacity of 8.61 mAh during 60°C discharge. Discharge

was carried out as follows. After charging of the cell had been completed, a constant temperature chamber was set to one of the discharge test temperatures. Once the constant temperature chamber reached the selected temperature, the cell was placed in the chamber for 6 hours. FIG. 7 shows the discharge curves obtained as a result of this test.

Example 14 [Secondary Cell 6]

Aside from the use of an electrolyte solution prepared by dissolving 17 parts by weight of lithium bis(trifluoromethanesulfonyl)imide in 83 parts by weight of the ionic liquid obtained in Synthesis Example 1 then adding 10 parts by weight of vinylene carbonate, a secondary cell was produced in the same way as in Example 13.

Example 15 [Secondary Cell 7]

Aside from the use of an electrolyte solution prepared by dissolving 35 parts by weight of lithium bis(trifluoromethanesulfonyl)imide in 65 parts by weight of the ionic liquid obtained in Synthesis Example 1 then adding 10 parts by weight of vinylene carbonate, a secondary cell was produced in the same way as in Example 13.

Example 16 [Secondary Cell 8]

Aside from the use of an electrolyte solution prepared by dissolving 39 parts by weight of lithium bis(trifluoromethanesulfonyl)imide in 61 parts by weight of the ionic liquid obtained in Synthesis Example 1 then adding 10 parts by weight of vinylene carbonate, a secondary cell was produced in the same way as in Example 13.

Example 17 [Secondary Cell 9]

Aside from using a polyolefin porous film (E25MMS, made by Tonen Tapyrus Co., Ltd.) as the separator, a secondary cell was produced in the same way as in Example 13.

Example 18 [Secondary Cell 10]

Aside from using an electrolyte solution prepared by adding 40 parts by weight of ethylene carbonate instead of 10 parts by weight of vinylene carbonate, a secondary cell was produced in the same way as in Example 13.

Example 19 [Secondary Cell 11]

Aside from using an electrolyte solution prepared by adding 10 parts by weight of vinylene carbonate and 30 parts by weight of ethylene carbonate instead of 10 parts by weight of vinylene carbonate, a secondary cell was produced in the same way as in Example 13. FIG. 8 shows the discharge curves obtained in load characteristics tests conducted on the resulting secondary cell.

Example 20 [Secondary Cell 12]

Aside from the use of an electrolyte solution prepared by dissolving 14.5 part by weight of lithium bis(trifluoromethanesulfonyl)imide and 14.5 parts by weight of lithium hexafluorophosphate in 71 parts by weight of the ionic liquid obtained in Synthesis Example 1, then adding 10 parts by weight of vinylene carbonate, a secondary cell was produced in the same way as in Example 13.

Example 21 [Secondary Cell 13]

Aside from using an electrolyte solution prepared by adding 10 parts by weight of vinylene carbonate and 15 parts by weight of ethylene carbonate instead of 10 parts by weight of vinylene carbonate, a secondary cell was produced in the same way as in Example 13.

The cells produced in above Examples 14 to 21 were subjected to initial performance tests, load characteristics tests and cycle tests under the same conditions as in Example 13. The results obtained from these tests are shown below in Table 4.

Table 4

| | Compound 8/Li salt (weight ratio) | Lithium salt | Compound which reductively decomposes | Content of compound which reductively decomposes (wt%) | Separator | Initial discharge capacity (mAh) | Capacity based $LiCoO_2$ (mAh/g) | Load characteristics (1C vs. 0.1C) (%) | Capacity retention in 100th cycle (during charging/ discharging at 0.1C) (%) |
|---|---|---|---|---|---|---|---|---|---|
| Example 13 | 71:29 | LiTFSI | VC | 10 | cellulose | 11.2 | 132.6 | 58.4 | 96.9 |
| Example 14 | 83:17 | LiTFSI | VC | 10 | cellulose | 10.8 | 127.6 | 55.7 | 95.2 |
| Example 15 | 65:35 | LiTFSI | VC | 10 | cellulose | 11.1 | 131.0 | 55.5 | 97.3 |
| Example 16 | 61:39 | LiTFSI | VC | 10 | cellulose | 11.2 | 133.1 | 72.4 | 97.0 |
| Example 17 | 71:29 | LiTFSI | VC | 10 | polyolefin porous film | 10.8 | 128.1 | 32.8 | 94.6 |
| Example 18 | 71:29 | LiTFSI | EC | 40 | cellulose | 11.2 | 132.1 | 95.2 | 97.4 |
| Example 19 | 71:29 | LiTFSI | VC/EC = 1/3 (by weight) | 40 | cellulose | 11.2 | 132.3 | 96.7 | 97.7 |
| Example 20 | 71:29 | LiTFSI/$LiPF_6$ = 1/1 (by weight) | EC | 40 | cellulose | 11.2 | 132.5 | 96.4 | 97.6 |
| Example 21 | 71:29 | LiTFSI | VC/EC = 1/1.5 (by weight) | 25 | cellulose | 11.2 | 132.0 | 88.5 | 97.2 |

VC: vinylene carbonate; EC: ethylene carbonate

**Claims**

1. A nonaqueous electrolyte **characterized by** containing:

an ionic liquid having general formula (1) below and a melting point not higher than 50°C

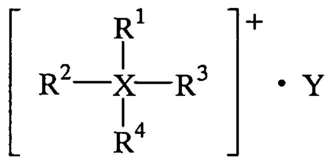

(1)

wherein $R^1$ to $R^4$ are each independently an alkyl group of 1 to 5 carbons or an alkoxyalkyl group of the formula $R'-O-(CH_2)_n-$ (R' being methyl or ethyl, and the letter n being an integer from 1 to 4), and any two from among $R^1$, $R^2$, $R^3$ and $R^4$ may together form a ring, with the proviso that at least one of $R^1$ to $R^4$ is an alkoxyalkyl group of the above formula,
X is a nitrogen atom or a phosphorus atom, and
Y is a monovalent anion;
a compound which reductively decomposes at a more noble potential than the ionic liquid; and
a lithium salt.

2. The nonaqueous electrolyte of claim 1 which is **characterized in that** the compound reductively decomposes at a more noble potential than the ionic liquid when a working electrode used with the electrolyte is made of a carbonaceous material or metallic lithium.

3. The nonaqueous electrolyte of claim 1 or 2 which is **characterized in that** the content of said compound within the electrolyte is from 0.1 to 60 wt%.

4. The nonaqueous electrolyte of claim 3 which is **characterized in that** the content of said compound is 0.1 to 30 wt%.

5. The nonaqueous electrolyte of any one of claims 1 to 4 which is **characterized in that** the compound is one or more selected from among ethylene carbonate, propylene carbonate, vinylene carbonate, dimethyl carbonate, ethyl methyl carbonate and diethyl carbonate.

6. The nonaqueous electrolyte of any one of claims 1 to 5 which is **characterized in that** the ionic liquid has a melting point not higher than 25°C.

7. The nonaqueous electrolyte of any one of claims 1 to 6 which is **characterized in that** X is a nitrogen atom, R' is methyl, and the letter n is 2.

8. The nonaqueous electrolyte of any one of claims 1 to 6 which is **characterized in that** the ionic liquid has general formula (2) below

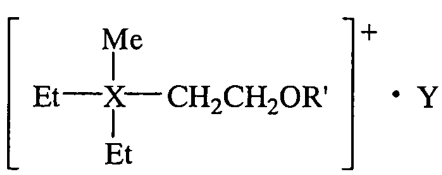

(2)

wherein R' is methyl or ethyl, X is a nitrogen atom or a phosphorus atom, Y is a monovalent anion, Me stands for methyl and Et stands for ethyl.

9. The nonaqueous electrolyte of any one of claims 1 to 8 which is **characterized in that** Y is $BF_4^-$, $PF_6^-$, $(CF_3SO_2)_2N^-$, $CF_3SO_3^-$ or $CF_3CO_2^-$.

**10.** The nonaqueous electrolyte of any one of claims 1 to 9 which is **characterized in that** the lithium salt is $LiBF_4$, $LiPF_6$, $Li(CF_3SO_2)_2N$, $LiCF_3SO_3$ or $LiCF_3CO_2$.

**11.** A nonaqueous electrolyte secondary cell having a positive electrode which contains a lithium-containing double oxide, a negative electrode which contains a carbonaceous material capable of inserting and extracting lithium ions or contains metallic lithium, a separator between the positive and negative electrodes, and a nonaqueous electrolyte;

which secondary cell is **characterized in that** the nonaqueous electrolyte is a nonaqueous electrolyte according to any one of claims 1 to 10.

**12.** The nonaqueous electrolyte secondary cell of claim 11 which is **characterized in that** the separator is a porous film or porous sheet having a thickness of 20 to 50 μm and a porosity of 25 to 85%.

**13.** The nonaqueous electrolyte secondary cell of claim 12 which is **characterized in that** the porous film or porous sheet is composed primarily of cellulose.

FIG.1

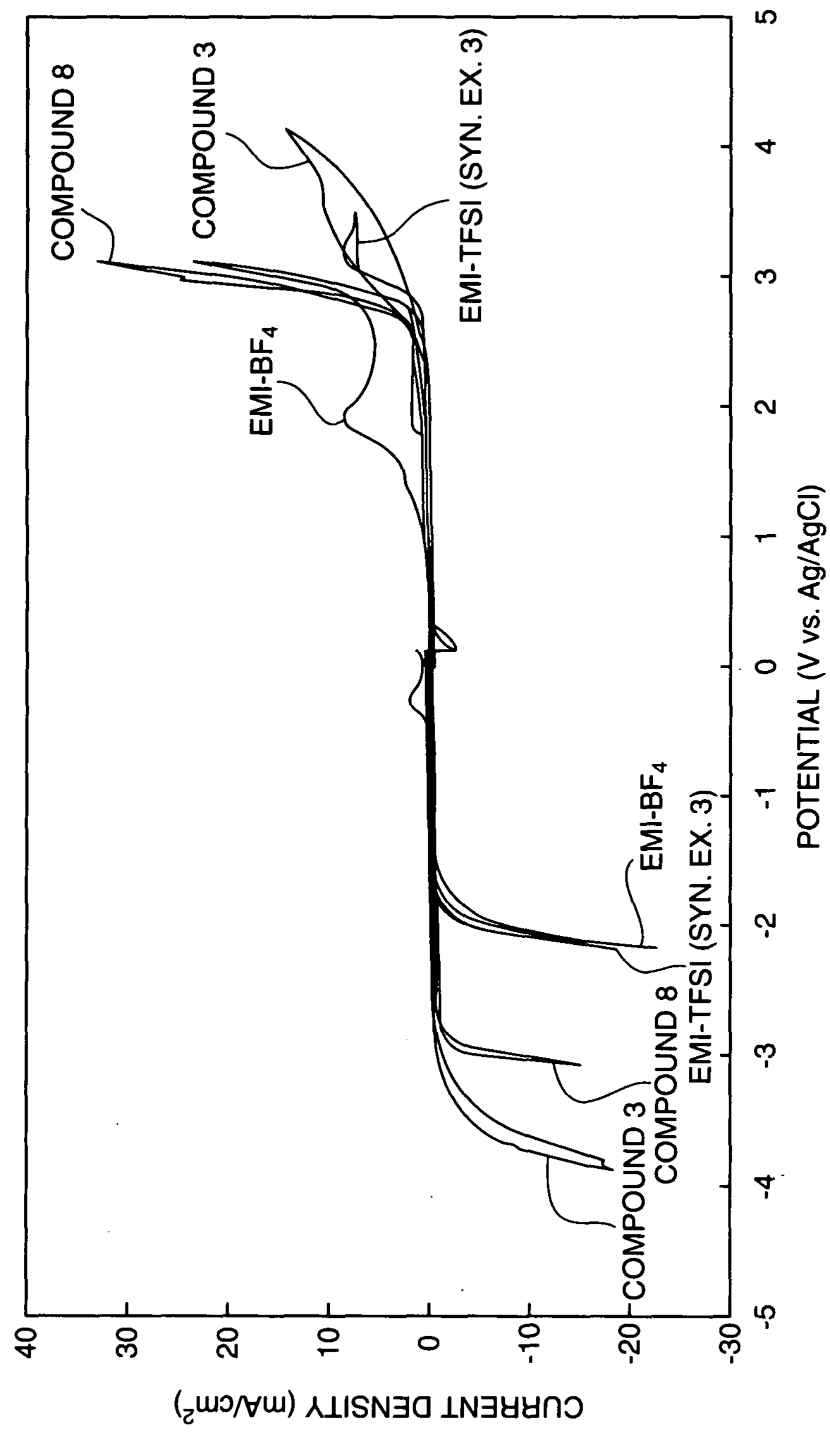
COMPOUND 8
COMPOUND 3
EMI-TFSI (SYN. EX. 3)
EMI-BF4
EMI-BF4
EMI-TFSI (SYN. EX. 3)
COMPOUND 8
COMPOUND 3
COMPOUND 3
40
30
20
10
0
-10
-20
-30
CURRENT DENSITY (mA/cm2)
-5
-4
-3
-2
-1
0
1
2
3
4
5
POTENTIAL (V vs. Ag/AgCl)

FIG.2

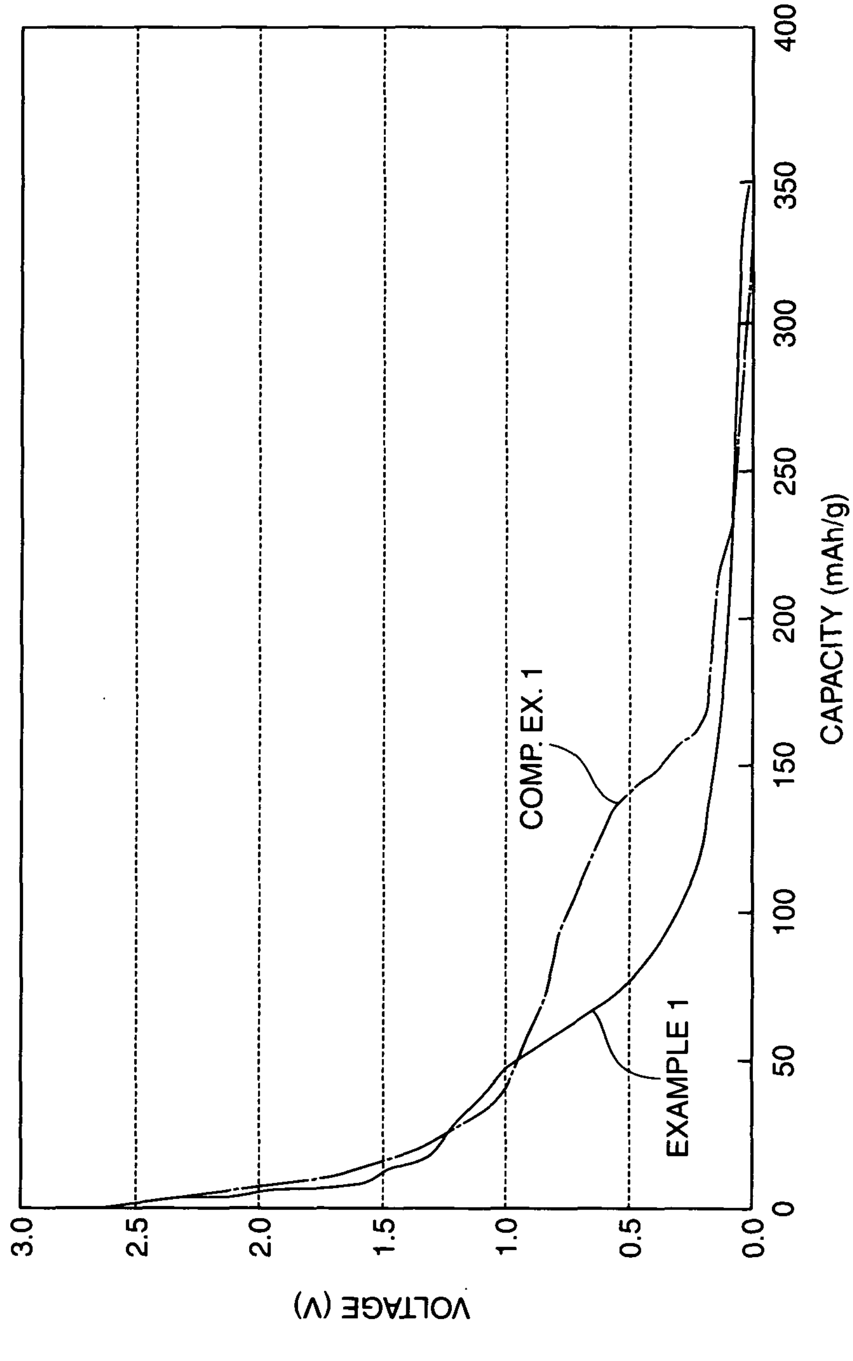
VOLTAGE (V)
3.0
2.5
2.0
1.5
1.0
0.5
0.0
CAPACITY (mAh/g)
0
50
100
150
200
250
300
350
400
COMP. EX. 1
EXAMPLE 1

FIG.3

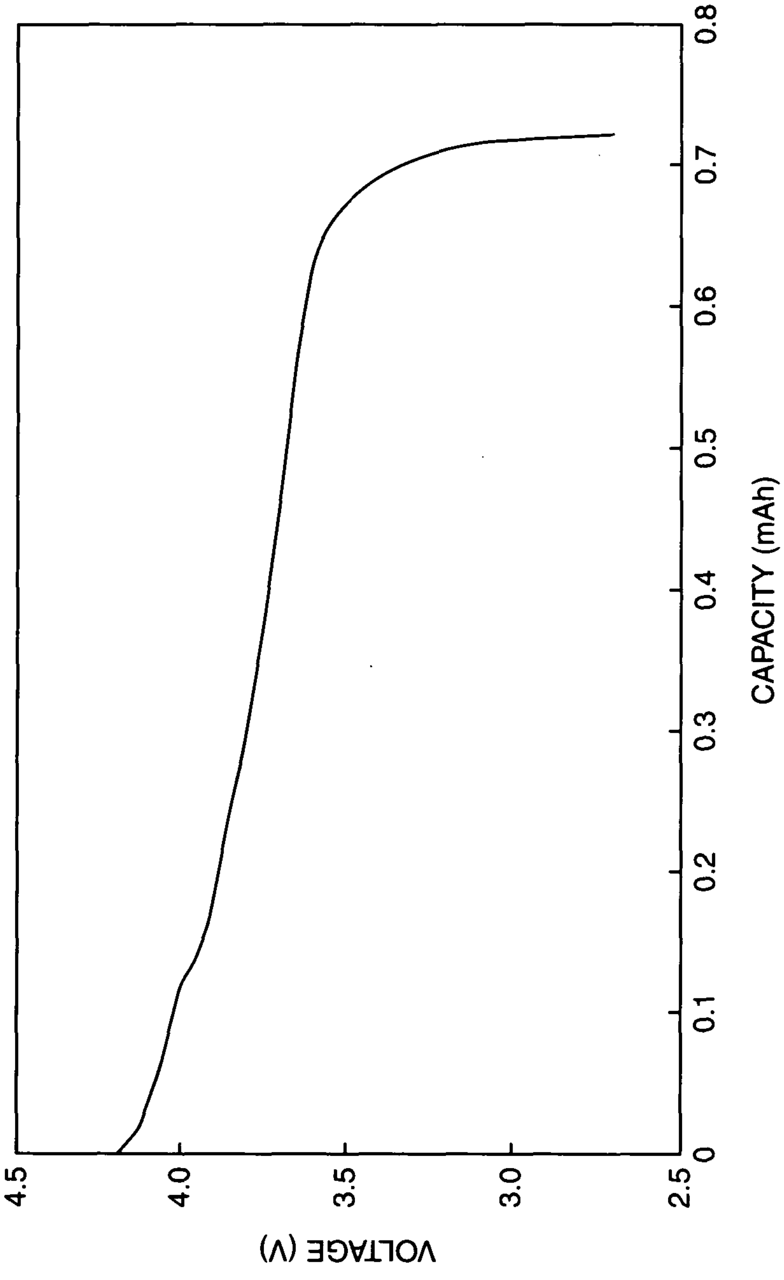

FIG.4

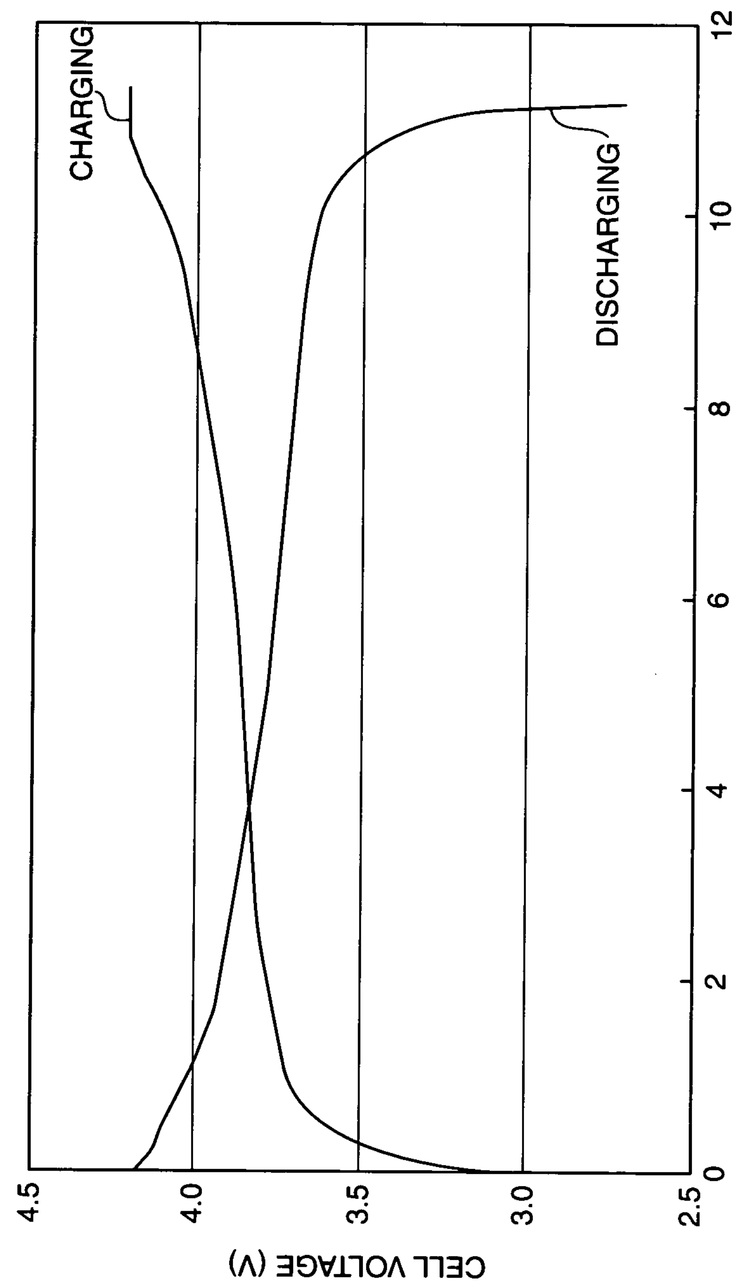
CHARGING
DISCHARGING
CELL VOLTAGE (V)
4.5
4.0
3.5
3.0
2.5
CAPACITY (mAh)
0
2
4
6
8
10
12

FIG.5

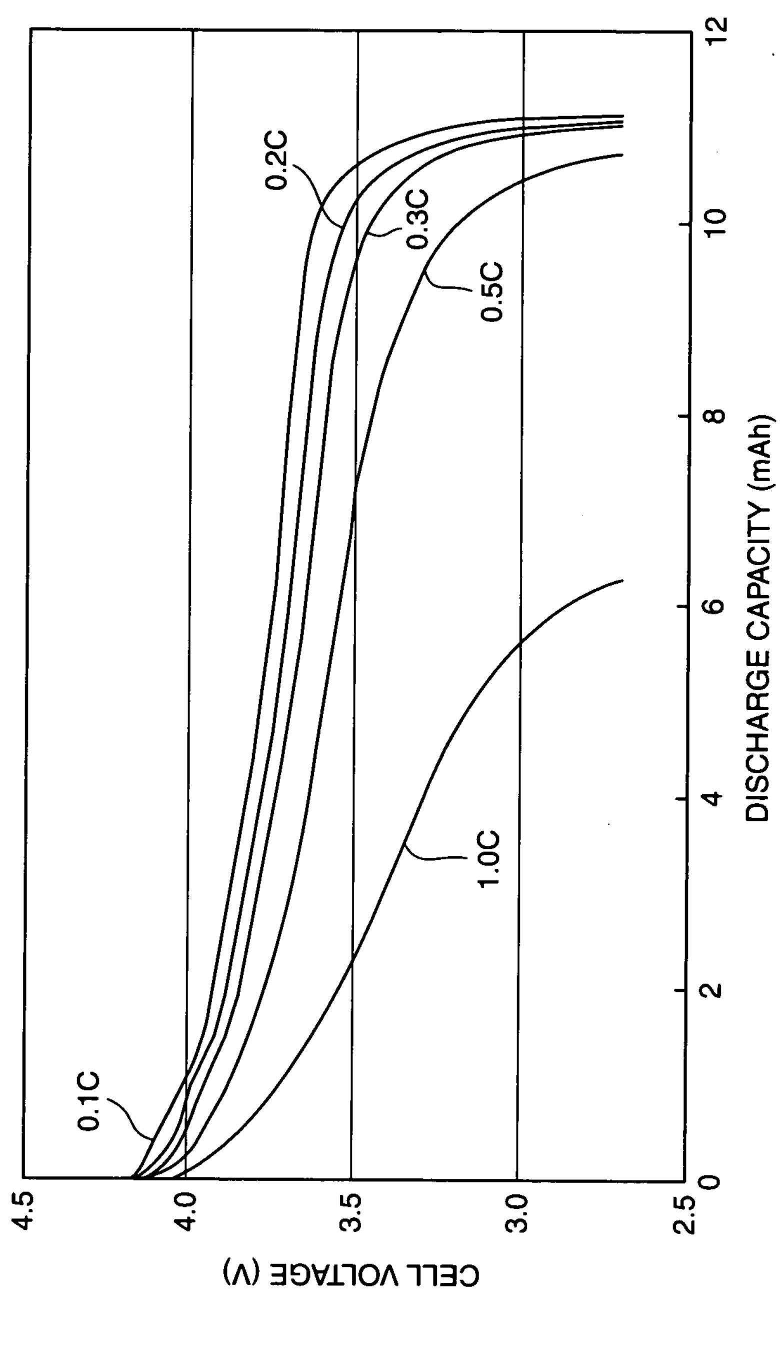
CELL VOLTAGE (V)
4.5
4.0
3.5
3.0
2.5
DISCHARGE CAPACITY (mAh)
0
2
4
6
8
10
12
0.1C
0.2C
0.3C
0.5C
1.0C

FIG.6

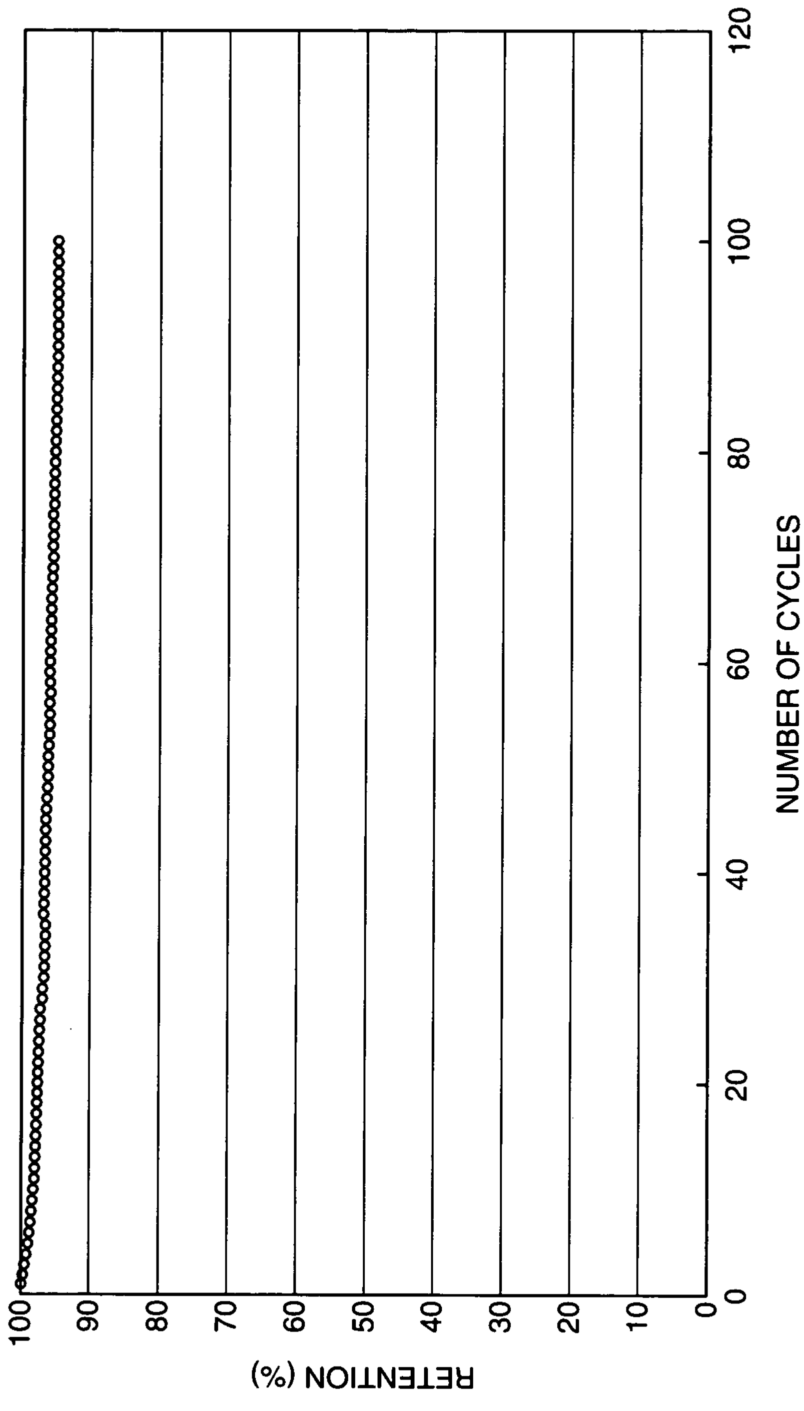
RETENTION (%)
100
90
80
70
60
50
40
30
20
10
0
NUMBER OF CYCLES
0
20
40
60
80
100
120

FIG.7

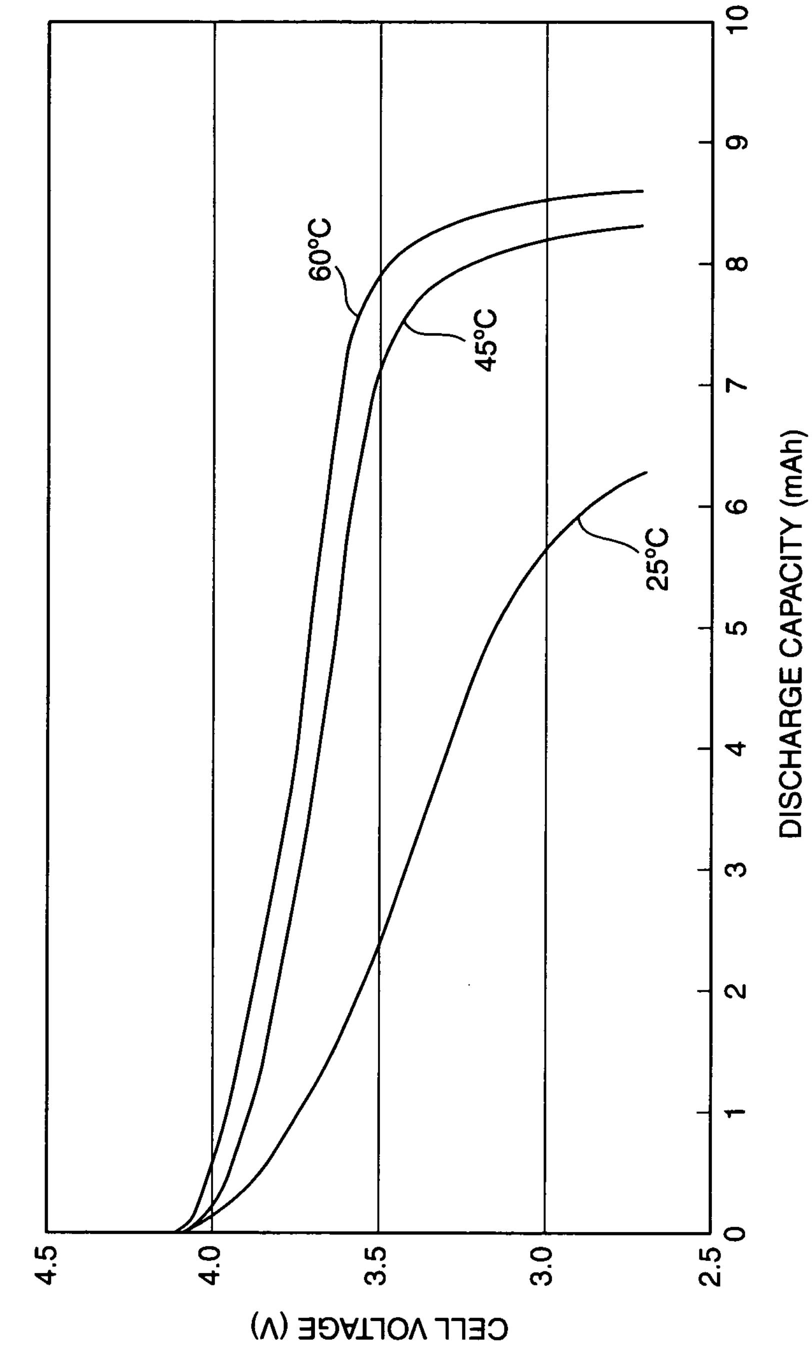
CELL VOLTAGE (V)
4.5
4.0
3.5
3.0
2.5
DISCHARGE CAPACITY (mAh)
0
1
2
3
4
5
6
7
8
9
10
60°C
45°C
25°C

FIG.8

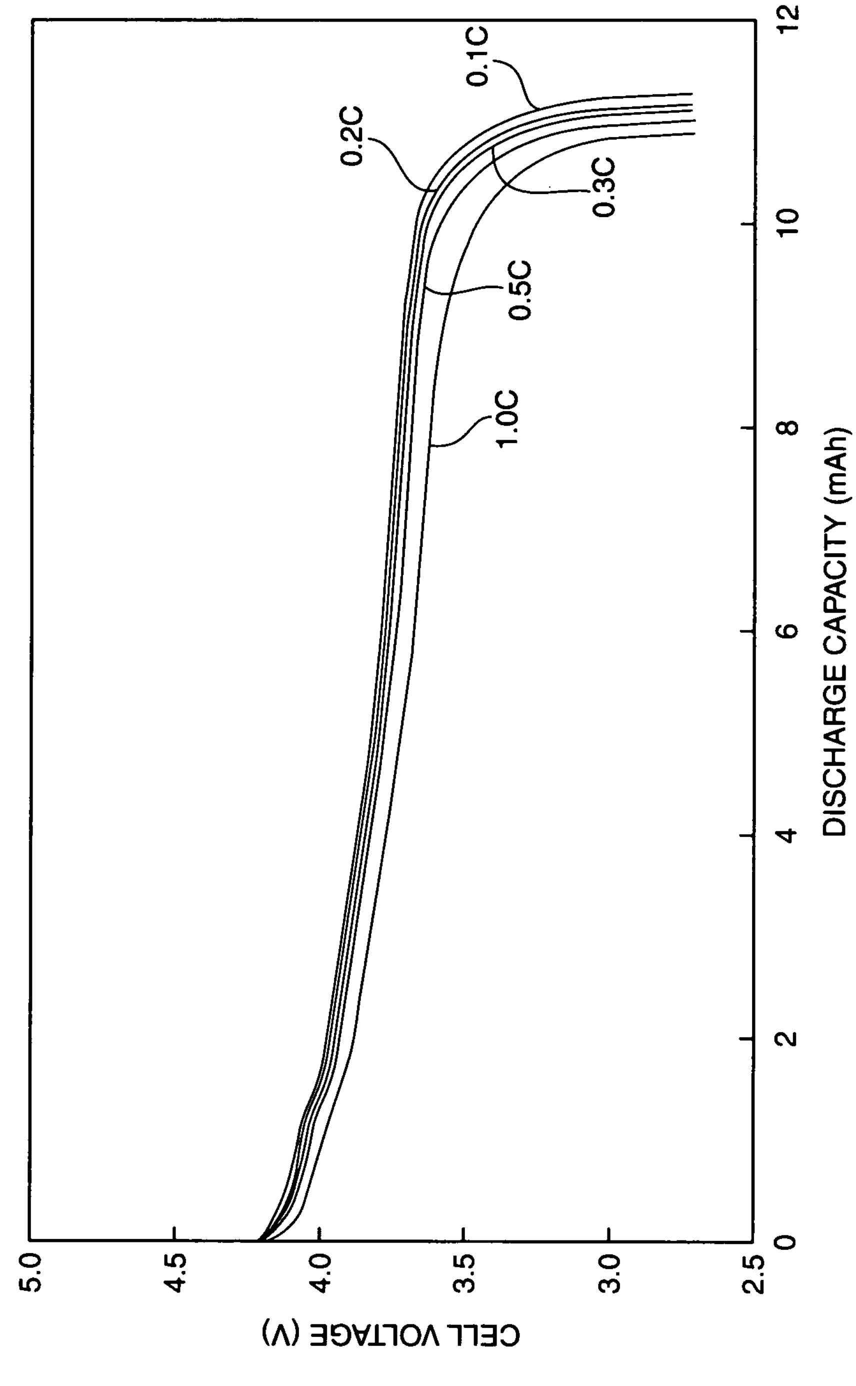
CELL VOLTAGE (V)
5.0
4.5
4.0
3.5
3.0
2.5
DISCHARGE CAPACITY (mAh)
0
2
4
6
8
10
12
0.1C
0.2C
0.3C
0.5C
1.0C

## INTERNATIONAL SEARCH REPORT

International application No.
PCT/JP03/10629

A. CLASSIFICATION OF SUBJECT MATTER
Int.Cl[7] H01M10/40, H01M2/16, C07C217/08, C07C311/48, C07D233/56

According to International Patent Classification (IPC) or to both national classification and IPC

B. FIELDS SEARCHED

Minimum documentation searched (classification system followed by classification symbols)
Int.Cl[7] H01M10/40, H01M2/16, C07C217/08, C07C311/48, C07D233/56

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 10-92467 A (Toshiba Corp.),<br>10 April, 1998 (10.04.98),<br>Claims; Par. Nos. [0010], [0014] to [0018], [0036]<br>(Family: none) | 1,5-10,12<br>2-4,11,13 |
| Y<br>A | JP 4-349365 A (Toshiba Corp.),<br>03 December, 1992 (03.12.92),<br>Claims; Par. Nos. [0010] to [0016], [0023]<br>(Family: none) | 1-2,5-12<br>3-4,13 |
| Y | JP 11-260400 A (Toshiba Corp.),<br>24 September, 1999 (24.09.99),<br>Claims<br>(Family: none) | 1-2,5-12 |

[x] Further documents are listed in the continuation of Box C.  [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 October, 2003 (24.10.03) | Date of mailing of the international search report<br>04 November, 2003 (04.11.03) |
| Name and mailing address of the ISA/<br>Japanese Patent Office<br><br>Facsimile No. | Authorized officer<br><br>Telephone No. |

Form PCT/ISA/210 (second sheet) (July 1998)

**INTERNATIONAL SEARCH REPORT**

International application No.
PCT/JP03/10629

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-307121 A (Mitsubishi Chemical Corp.), 05 November, 1999 (05.11.99), Claims (Family: none) | 1-2,5-12 |
| Y | JP 9-92257 A (Sony Corp.), 04 April, 1997 (04.04.97), Claims (Family: none) | 12 |
| Y | JP 11-67273 A (Toshiba Corp.), 09 March, 1999 (09.03.99), Claims (Family: none) | 12 |
| P,X<br>P,A | WO 02/076924 A1 (Nisshinbo Industries, Inc.), 03 October, 2002 (03.10.02), Claims; page 14, line 19 to page 17, line 28; page 34, line 16 to page 35 line 1; page 36, lines 22 to 28; page 37, lines 18 to 21 (Family: none) | 1-2,5-13<br>3-4 |

Form PCT/ISA/210 (continuation of second sheet) (July 1998)